# EUROPEAN PATENT SPECIFICATION

(11) **EP 3 672 625 B1**
(45) Date of publication and mention of the grant of the patent: **12.07.2023**
(21) Application number: 18755823.4
(22) Date of filing: 20.08.2018
(51) Int. Cl.: A61K 39/00

(54) **SYNTHETIC VACCINE**
SYNTHETISCHER IMPFSTOFF
VACCIN SYNTHÉTIQUE

(30) Priority: 23.08.2017 EP 17187600
(43) Date of publication of application: 01.07.2020
(73) Proprietor: Medizinische Hochschule Hannover, 30625 Hannover (DE)
(72) Inventor: WIRTH, Thomas, 31319 Sehnde (DE); SLÜTTER, Bram, 2333 CC Leiden (NL)
(74) Representative: Taruttis, Stefan Georg
(86) International application number: PCT/EP2018/072433
(87) International publication number: WO 2019/038230

(56) References cited:
- WO-A1-2010/054654
- N.-L. L. PHAM ET AL: "Exploiting cross-priming to generate protective CD8 T-cell immunity rapidly", PROCEEDINGS OF THE NATIONAL ACADEMY OF SCIENCES, vol. 107, no. 27, 6 July 2010 (2010-07-06) , pages 12198-12203, XP055159169, ISSN: 0027-8424, DOI: 10.1073/pnas.1004661107 cited in the application
- SEYED AMIR JALALI ET AL: "Induction of tumor-specific immunity by multi-epitope rat HER2/neu-derived peptides encapsulated in LPD Nanoparticles", NANOMEDICINE: NANOTECHNOLOGY, BIOLOGY AND MEDICINE, vol. 8, no. 5, 1 July 2012 (2012-07-01), pages 692-701, XP055415717, NL ISSN: 1549-9634, DOI: 10.1016/j.nano.2011.09.010

## Description

The invention is set out in the appended set of claims. The embodiments and/or examples of the following description which are not covered by the appended claims are considered as not being part of the present invention. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention relates to a pharmaceutical combination of compositions for use in the treatment or prevention of a disease having cells bearing a target antigen, e.g. as a vaccine for vaccination of a mammal, especially of a human for raising a cellular and/or a humoral immune response directed against cells of the mammalian recipient of the combination of compositions, especially human recipient, which cells express a target antigen. The target antigen can e.g. be an autoantigen like a malignant antigen, i.e. a tumour-specific antigen, e.g. a neo-antigen, or an alloantigen specific for an infecting agent, e.g. an antigen specific for a virus or for an intracellular bacterium. Preferably, the pharmaceutical combination of compositions and the method are for medical use in the treatment of tumour and/or for medical use in the treatment of infections by a virus or by intracellular bacteria.

The pharmaceutical combination of compositions is synthetic, i.e. consists of ingredients produced in vitro and is free from cells, especially free from dendritic cells. The pharmaceutical combination of compositions comprises or consists of a first composition and a second composition, wherein the second composition is for administration to the mammalian, especially human recipient, subsequent to the administration of the first composition, e.g. the second composition is provided for administration at least 2 days or at least 3 days, preferably at least 5 days, e.g. at 7 or 10 days or later following administration of the first composition. Accordingly, a method of medical treatment comprises the administration of the first composition and the subsequent administration of the second composition to a recipient, e.g. at least 2 days or at least 3 days, e.g. at 5 to 10 days or later subsequent to administration of the first composition. The method of treatment is effective in inducing an antigen-specific T-cell response in the recipient, preferably in combination with an antibody response, wherein the T-cell response and/or antibody response is directed against cells bearing the target antigen, which preferably is an auto-antigen like a tumour-antigen. The pharmaceutical combination of compositions for use in treatment has the advantage of raising an effective antigen-specific T-cell response and/or an antibody response against cells bearing a target antigen that can be an alloantigen or an autoantigen, especially against a target antigen which is a malignant autoantigen, e.g. raising an antigen-specific T-cell response against cells bearing a tumour-antigen. A further advantage is that the pharmaceutical combination of compositions can raise an antigen-specific T-cell response within a comparatively short time, e.g. within 3 to 14 days, e.g. 5 to 10 days after administration of the first composition. The antigen-specific T-cell response is a CD8+ T-cell response and/or a CD4+ T-cell response, preferably a CD8+ T-cell response in combination with a CD4+ T-cell response. The pharmaceutical combination of compositions has the advantage that it does not contain immune cells, e.g. no dendritic cells (DC), originating from the later recipient of the pharmaceutical combination of compositions.

### State of art

US 2003/0077263 A1 describes the in vitro production of antigen-presenting dendritic cells for use as a vaccine adjuvant against tumour. CD34+ hematopoietic progenitor cells and stem cells were stimulated with granulocyte-macrophage colony stimulating factor (GM-CSF) for in vitro expansion and differentiation into dendritic cells that were contacted with an antigen or transfected with a gene encoding the antigen, and subsequently activated with a CD40-binding protein. These antigen-pulsed dendritic cells were reintroduced into the original donor of the CD34+ cells.

WO 2010/054654 A1 describes administration of the same cationic liposomes comprising neutral lipids and an antigen three subsequent times with a delay of two weeks between injections.

Jalali et al., Nanomedicine: Nanotechnology, Biology, and Medicine 692-701 (2012) describes the administration of three consecutive vaccinations at a two week interval of antigenic peptide encapsulated in liposomes.

US 2006/0204509 A1 describes immunization of mice with dendritic cells coated with a peptide representing an epitope of Listerium monocytogenes, followed by a booster immunization with complete Listerium monocytogenes bacteria.

Ahonen et al., J. Exp. Med. 775-784 (2004) describe the immunization of naive mice using concomitant administration of the alloantigen ovalbumin or its epitope peptide SIINFEKL with a TLR agonist and anti-CD40 antibody. The combination of the TLR agonist and anti-CD40 antibody with the antigen was found to induce expansion of antigen-specific CD8+ T-cells. No booster immunization is described.

Poly(I:C) (polyinosinic:polycytidylic acid) is a mismatched double-stranded RNA, one strand being comprised of polyinosinic acid, the other strand of polycytidylic acid. Poly(I:C) is known to interact with TLR3 (Toll-like receptor 3) and is used as an immuno stimulant, e.g. using the sodium salt of Poly(I:C) for simulating viral infections.

Ricupito et al., Cancer Research 3545-3554 (2013) describe a vaccine comprising as a first component antigen-pulsed dendritic cells (DC), and for subsequent administration as a boost a second component of the antigen in complete Freunds adjuvant. The antigen was Tag-IV, the immunodominant CTL epitope from the SV40 Tag antigen. Ricupito et al. conclude that a single administration of the antigen-primed DC is effective against tumours, whereas the boost does not sustain survival of tumour-specific T_{CM} cells and is rather detrimental to long-lived immune surveillance.

Badovinac et al., Nature Medicine, 748-756 (2005) describe a first composition which can be DC primed with the antigen LLO of Listerium monocytogenes, vaccinia virus expressing the LLO91-99 epitope of Listerium monocytogenes or syngeneic spleen cells coated with the LLO91-99 epitope of Listerium monocytogenes, and a second composition for boosting, consisting of virulent Listerium monocytogenes.

Pham et al., PNAS 12198-12203 (2010) describe immunisation using antigen-coated synthetic PLGA microspheres to replace antigen-coated DC as a first component of a vaccine, using hen ovalbumin (Ova) as the antigen. Following administration of the first component consisting of antigen-coated PLGA microspheres, a second component of virulent Listerium monocytogenes expressing Ova (virLM-Ova), or of full-length Ova-protein plus poly(I:C) plus anti-CD40 mAb was administered for boosting. Pham et al. conclude that the effect of the boost is based on the cross-priming against particulate antigen, because immunisation with twice the amount of soluble Ova did not prime a boostable CD8 T-cell response.

US 2011/0274653 A1 describes a conjugate of an anti-CD40 antibody and an antigen for immunisation in a composition containing a TLR agonist and optionally an anti-CD40 antibody. No boosting composition for subsequent administration is mentioned.

WO 2012/135132 A1 describes a fusion peptide of an antibody specific for a DC specific cell surface receptor and a HCV antigen, which optionally is used in combination with a TLR agonist. No boosting composition for subsequent administration is mentioned.

Capece et al., J. of Biomedicine and Biotechnology 1-17 (2012) describe various costimulatory and co-inhibitory pathways affecting anti-tumour immune responses and anergy of T-cells against tumour antigens.

Yan Ge et al., Biomedicine and Pharmacotherapy 487-492 (2010) describe immunisation by administration of immature DC and the agonistic anti-CD40 antibody 5C11.

Lapteva et al., Cancer Res. 10548-10537 (2008) describes improving vaccines based on DC by use of a fusion peptide of the cytoplasmatic domain of anti-CD40 and a synthetic ligand binding domain with a membrane targeting sequence for inducing the CD40 signal cascade in DC.

WO 2015/165997 A1 describes a vaccine composed of a first composition containing DC associated with a target antigen and a second composition for administration a few days subsequent to administration of the first composition, the second composition containing the target antigen, a co-stimulatory antibody for activating T-cells and/or DC, and Poly(I:C)

### Object of the invention

It is an object of the invention to provide pharmaceutical compositions suitable for effectively raising an immune response, preferably a cellular immune response, especially a CD8+ T-cell response, against cells of a human recipient, which cells bear a target antigen, which preferably is a malignant antigen, e.g. a tumour antigen, preferably raising a CD8+ T-cell response against cancer.

### Description of the invention

The invention achieves the object by the features of the claims, especially by providing a pharmaceutical combination of compounds, which are provided in a first composition of compounds and in a second composition of compounds, for medical use in the treatment or prevention especially of tumours or of infections by virus or intracellular bacteria. The pharmaceutical combination of compounds is provided for administration to a mammal, preferably a human, herein also referred to as a recipient, preferably a human recipient. The first composition is prepared for first administration and the second composition is prepared for separate administration subsequent to administration of the first composition, e.g. for administration subsequent to administration of the first composition by at least 1 to at least 10 days, e.g. subsequent by at least 2, e.g. to at least 7 days, preferably subsequent by at least 3 days or by at least 5 days, e.g. 2 or 3 to at least 7 days, or later, subsequent to administration of the first composition. Preferably, the second composition is for administration 2 days later or 3 days or later subsequent to administration of the first composition. The pharmaceutical combination of compounds, which are comprised in the first and second compositions, is adapted for eliciting a target antigen-specific CD8+ T-cell response, preferably including a target antigen-specific CD4+ T-cell response, for the prevention and/or treatment of cells bearing the target antigen, which preferably is a malignant antigen, e.g. an autologous tumour antigen for the prevention and/or treatment of tumours bearing tumour antigen. Alternatively, the target antigen is an alloantigen, e.g. an antigen caused by infection by intracellular pathogens, e.g. infections by intracellular bacteria or viral infections and the pharmaceutical combination of compounds is for use in the prevention and/or treatment of infections by a virus or by intracellular bacteria. Accordingly, the pharmaceutical combination is customized for vaccination or for the prevention and/or treatment of tumours or for the prevention and/or treatment of these infections. Generally, the first composition can also be termed a first component and the second composition can also be termed a second component, and the combination comprising or consisting of the first and second component can be termed a medicament or vaccine. Accordingly, the pharmaceutical combination of compositions for use in medical treatment comprises the combination of a first composition comprising liposomes containing a target antigen, and a second composition comprising at least a portion of the target antigen, e.g. the same target antigen, in soluble form and a co-stimulatory antibody effective for activating T-cells, wherein the second composition is for administration at a time at least 1 day, preferably at least 2 days or at least 3 days or at least 7 days, e.g. 5 to 7 days subsequent to administration of the first composition. Generally, the liposomes can be neutral or anionic liposomes, preferably the liposomes are cationic liposomes. The liposomes, which preferably are cationic liposomes, are formulated for uptake by DC, e.g. when the liposomes are administered systemically, e.g. by injection. The first composition can optionally comprise an adjuvant, or the first composition can be devoid of an adjuvant. Optionally, the liposomes can contain an adjuvant, arranged on the outside of the liposomes and/or within the liposomes only. The adjuvant can e.g. be selected from the group consisting of Toll like receptor agonists, NOD like receptor agonists, RIG-I like receptor agonists and STING pathway agonists (STING agonists), and mixtures of at least two of these. Exemplary STING pathway agonists are e.g. cyclic dinucleotides, for example cdi-GMP, or synthetic compounds, e.g. MK1454 or ADU-S100.

Generally, the combination of compositions, respectively, can be for neoadjuvant use, e.g. for reduction of the tumour prior to surgery, and/or for adjuvant use, e.g. following tumour surgery, or for palliative use, e.g. without tumour surgery.

The pharmaceutical combination of compounds can be provided for use in the treatment of tumours that can be selected from the group comprising solid cancers and haematological malignancies, e.g. selected from the group comprising or consisting of haematological malignancies, e.g. Hodgkin and non-Hodgkin lymphomas, leukemia, e.g. acute lymphoblastic leukemia, acute myeloid leukemia, chronic lymphocytic leukemia, chronic myeloid leukemia, monocytic leukemia, myelomas, myeloproliferative diseases, myelodysplastic syndromes and solid cancers, e.g. originating from brain, head and neck, lung, pleura, heart, liver, kidney, colon, pancreas, stomach, gut, urinary tract, prostate, uterus, ovaries, breast, skin, testes, larynx and sarcoma.

Further, there is described a method of treatment, raising a target antigen specific immune response, especially a cellular immune response specifically directed against cells bearing a target antigen, which especially is a malignant or a tumour antigen, preferably a homologous antigen, e.g. human tumour antigen, or a viral antigen or an intracellular bacterial antigen, by administration of the components comprised in the first composition and in the second composition of the pharmaceutical combination to a recipient, wherein the second composition is for administration subsequent to administration of the first composition.

The first composition of the pharmaceutical combination is customised for priming a target antigen-specific CD8+ T-cell response, preferably inducing the generation of target antigen-specific memory T-cells, and the second composition is customised for boosting the target antigen-specific CD8+ T-cell response. It has been found that the second composition can be customised for administration 2, preferably 3 days, e.g. up to 10 days, e.g. at 3 days or 5 days to 7 days following administration of the first composition for generating an important antigen-specific CD8+ T-cell response or cell number. Currently it is assumed that target antigen-specific memory T-cells, especially those specific for a target antigen that is a tumour antigen, are induced within at maximum 10, preferably at maximum within 7 days following administration of the first composition, and accordingly, the memory T-cells induced by the administration of the combination of compounds of the invention can be described as early memory T-cells. Optionally, the first composition does not contain components which induce a systemic inflammation. Accordingly, the target antigen contained in the first composition preferably is an autoantigen, e.g. a tumour antigen. Optionally the first composition is free from adjuvants, especially free from adjuvants that stimulate a strong systemic immune response, and/or, optionally preferably, the first composition contains a compound effective for activating dendritic cells, which is an adjuvant that induces only a local activation of dendritic cells. Such a compound effective for activating dendritic cells can be selected from Toll like receptor (TLR) agonists, NOD like receptor agonists, RIG-I like receptor agonists and STING pathway agonists, and mixtures of at least two of these. It was found in exemplary tests that an effective induction of antigen-specific T-cells was generated in a mammal at 12 to 15 days after the administration of the first composition when the second composition was administered at 3 to 5 days, e.g. 7 days, subsequent to administration of the first composition.

Preferably, the immune response additionally induces target antigen-specific CD4+ T-cells that support B-cell mediated antibody production and tumour-specific Th1 T-cell responses.

Further, there is described a method for raising an antigen-specific T-cell response in a recipient against cells expressing a target antigen by administration of the pharmaceutical combination, firstly administration of the first composition, and subsequently of the second composition, with a temporal delay of at least 1 day, e.g. at least 2 or at least 3 days.

The first composition comprises or consists of liposomes, which preferably are cationic, containing the target antigen and optionally a compound effective for activating dendritic cells. The liposomes containing the target antigen preferably are unilamellar and have a size of 60 to 500 nm, e.g. 180 to 140 nm, preferably of 160 nm. Generally optionally, liposomes of the pharmaceutical combinations of compositions can be multilamellar. The liposomes can e.g. be prepared by preparing a lipid film of the lipid constituents, which preferably are cationic lipid constituents, e.g. by evaporating a solvent from the lipid constituents, followed by hydration of the lipid film using an aqueous preparation of the antigen, followed by lyophilisation and rehydration in an aqueous composition containing the antigen and optionally a compound effective for activating dendritic cells and/or an adjuvant, e.g. a TLR agonist, which compound preferably is selected from Toll like receptor agonists, NOD like receptor agonists, RIG-I like receptor agonists and STING pathway agonists or a mixture of at least two of these, to produce a suspension of multilamellar vesicles containing the antigen and optionally the compound effective for activating dendritic cells and/or the adjuvant, e.g. a TLR agonist, and pushing, e.g. by extrusion, the suspension of multilamellar vesicles through a membrane having pores to produce monodisperse unilamellar liposomes containing the antigen. These monodisperse unilamellar liposomes preferably have a diameter of 180 to 140 nm, preferably of 160 nm, e.g. using a membrane having pores of 200 nm. The liposomes contain cholesterol, preferably up to 10 % by weight, e.g. 2 to 10 % by weight, of lipids, and preferably are cationic due to the lipids containing or consisting of at least one cationic lipid, which is hydrophobic and has a net positive charge, preferably due to containing a quaternized amine group. The cationic lipid can be selected from phospholipids, triglycerides, ceramindes and sphingolipids, cholesterol and mixtures of at least two of these. Preferably, the cationic liposomes, in which the target antigen is contained, comprise or consist of 1,2-distearoyl-sn-glycero-3-phosphocholine (DSPC), 1,2-dipalmitoyl-3-trimethylammonium-propane (DPTAP) and cholesterol as the lipid constituents. The lipid constituents preferably are in a molar ratio of 4:1:2, each in a range of plus or minus 20 %, preferably each in a range of plus or minus 5% to 10%, more preferably in a molar ratio of 4:1:2, for 1,2-distearoyl-sn-glycero-3-phosphocholine (DSPC) : 1,2-dipalmitoyl-3-trimethylammonium-propane (DPTAP) : cholesterol.

The contained compound effective for activating dendritic cells and/or the adjuvant, e.g. a TLR agonist, can be bound to the outside surface of the liposomes, e.g. only to the outside surface of the liposomes, but preferably this compound is contained within the liposomes, optionally arranged within the liposomes only. Alternatively, this compound can be for systemic administration, e.g. for concomitant administration or later administration. Generally, the compounds of the first composition or the compounds of the second composition which are not arranged on the outside of liposomes and/or within liposomes are formulated for concomitant systemic administration, e.g. in combination with the liposomes containing the antigen in one liquid formulation, or as a separate formulation for systemic administration concomitant with the administration of the liposomes.

The target antigen is an antigen specific for the malignant cells within the recipient, e.g. selected from tumour-specific antigens (malignant antigens), viral antigens and antigens of intracellular bacteria. The target antigen can be an antigen presented by MHC class I or by MHC class II molecules.

The first composition can contain or consist of the liposomes which are loaded with a target antigen in a pharmaceutically acceptable formulation that is adapted to keep intact the antigen-loaded liposomes, e.g. an aqueous formulation, e.g. suspended in physiologic solution, e.g. in physiologic saline. Preferably, the first composition is a formulation adapted for intramuscular, sub-cutaneous, intra-venous or intraperitoneal administration.

The effective induction of a target antigen-specific immune response by the combination of the first composition and the second composition, which target antigen preferably is a tumour antigen, which is autologous, is surprising, because tumours generally evade the immune surveillance and express homologous antigen, against which generally immune responses of only very low magnitude can be elicited. Further, it is surprising that a high number of target antigen-specific T-cells can be induced by administration of the second composition within a short time subsequent to administration of the first composition. The short time delay between administration of the first and of the second compositions allow the use of the combination of the compositions for use in the treatment of tumours or in the treatment of infections by virus or intracellular bacteria, because no long time delay needs to occur before an effective target antigen-specific cellular immune response is induced.

The adjuvant can be selected from a Toll like receptor (TLR) agonist, a NOD like receptor agonist, a RIG-I like receptor agonist and a STING pathway agonist, and mixtures of at least two of these.

The TLR agonist can e.g. be selected from the group comprising or consisting of TLR1/2 agonists, e.g. lipoproteins and/or peptidoglycans, TLR3 agonists, e.g. natural or synthetic double-stranded RNA, e.g. Poly(I:C) or PolyICLC, TLR4 agonists, e.g. lipopolysaccharides, TLR5 agonists, e.g. flagellin, TLR6 agonists, e.g. diacetylated lipoprotein, TLR7 and TLR8 agonists, e.g. single-stranded RNA, TLR9 agonists, e.g. non-methylated and GpG-containing DNA, and/or TLR11 agonists, e.g. profilin, and combinations of at least two of these. The TLR agonist preferably is a TLR3 agonist, e.g. Poly(I:C) or PolyICLC (polyinosinic-polycytidylic acid stabilized with polylysine and carboxymethylcellulose, available under the trademark Hiltonol), a TLR7 agonist, a TLR4 agonist, a TLR9 agonist and combinations of at least two of these.

In the embodiments of the invention, the second composition comprises or consists of a target antigen and a co-stimulatory antibody for CD8+-T cells and/or for DC, and preferably an adjuvant, e.g. selected from a Toll like receptor (TLR) agonist, a NOD like receptor agonist, a RIG-I like receptor agonist and a STING pathway agonist, and mixtures of at least two of these, wherein the TLR agonist e.g. is a TLR3 agonist, e.g. Poly(I:C) or PolyICLC (polyinosinic-polycytidylic acid stabilized with polylysine and carboxymethylcellulose, available under the trademark Hiltonol), a TLR7 agonist, a TLR4 agonist, a TLR9 agonist and combinations of at least two of these, in a pharmaceutical formulation.

Optionally, at least one of the compounds of the second composition can be formulated in liposomes. Preferably, in the second composition, the target antigen and at least one co-stimulatory antibody and the optional adjuvant, e.g. the target antigen and the co-stimulatory antibody only are contained in liposomes and the optional adjuvant is in admixture, preferably aqueous admixture, with these liposomes, or the target antigen and the optional adjuvant only are contained in liposomes and the co-stimulatory antibody is in admixture, preferably aqueous admixture, with these liposomes, or only the target antigen is contained in liposomes and both the co-stimulatory antibody and the optional adjuvant are in admixture, preferably aqueous admixture, with these liposomes, or the target antigen and the co-stimulatory antibody and the optional adjuvant are contained in liposomes. In the second composition, the liposomes can be neutral or anionic, preferably cationic liposomes. Preferably, in the second composition, the liposomes are of the same lipid composition as the liposomes of the first composition. The liposomes of the first composition preferably are monodisperse unilamellar liposomes preferably having a diameter of 180 to 140 nm, preferably of 160 nm, e.g. using a membrane having pores of 200 nm. These liposomes contain cholesterol, preferably up to 10 % by weight, e.g. 2 to 10 % by weight, of lipids, and preferably are cationic due to the lipids containing or consisting of at least one cationic lipid, which is hydrophobic and has a net positive charge, preferably due to containing a quaternized amine group. The cationic lipid can be selected from phospholipids, triglycerides, ceramindes and sphingolipids, cholesterol and mixtures of at least two of these. Preferably, the cationic liposomes, in which the target antigen can be contained, comprise or consist of 1,2-distearoyl-sn-glycero-3-phosphocholine (DSPC), 1,2-dipalmitoyl-3-trimethylammonium-propane (DPTAP) and cholesterol as the lipid constituents. The lipid constituents preferably are in a molar ratio of 4:1:2, each in a range of plus or minus 20 %, preferably each in a range of plus or minus 5% to 10%, more preferably in a molar ratio of 4:1:2, for 1,2-distearoyl-sn-glycero-3-phosphocholine (DSPC) : 1,2-dipalmitoyl-3-trimethylammonium-propane (DPTAP) : cholesterol.

The co-stimulatory agonistic antibody for CD8+ T-cells and/or DC and/or the compound effective for activating DC is a molecule specifically directed against a surface receptor of T-cells and/or of DCs of the recipient and is selected from an anti-CD137 antibody, an anti-CD40 antibody, an anti-OX40 antibody, an anti-ICOS antibody, an anti-CD27 antibody, an anti-CD28 antibody, an anti-GITR antibody, specifically anti-human GITR/AITR antibody, an anti-HVEM antibody, an anti-TIM1 antibody, an anti-TIM3 antibody, and mixtures of at least two of these.

The second composition is a formulation for intramuscular, sub-cutaneous, intra-venous or intraperitoneal administration. Optionally, the second composition is provided for systemic administration. The second composition can be provided for administration, e.g. injection, at the same site or at a different site of the recipient's body.

The target antigens of the first composition and of the second composition contain at least one identical epitope for MHC I and/or for MHC II. Preferably, the malignant antigen of the first composition and the malignant antigen of the second composition share at least one section, the section having an identity of at least 80%, preferably for at least 90%, more preferably for at least 95% or at least 99% of the amino acid sequence. Optionally, the malignant antigens of the first composition and of the second composition are identical.

The target antigen, e.g. a malignant antigen, preferably is soluble in aqueous media, e.g. in medium suitable for hydration of a lipid film generated from the lipid constituents of the liposomes, and is soluble in the second composition. The antigen can be a proteinaceous antigen, i.e. a protein or peptide, or a nucleic acid, e.g. RNA, especially mRNA, DNA, especially cDNA or genomic DNA, encoding the proteinaceous antigen, e.g. with genetic elements directing the biosynthesis of the proteinaceous antigen from the encoding nucleic acid. Genetic elements directing the synthesis of a peptide encoded by a nucleic acid are generally known, e.g. ribosome binding sites, internal ribosomal entry sites (IRES), promotors and enhancers. Preferably, the target antigen is a proteinaceous antigen, e.g. a full-length protein of the natural amino acid sequence or a portion thereof comprising at least 8 or at least 12 amino acids. Preferably, the first composition and the second composition both contain the same target antigen. Generally, the tumour antigen can be an antigen that is re-expressed in tumour cells or an antigen that is overexpressed in tumour cells, e.g. in comparison to differentiated normal cells. Exemplary tumour antigens of humans are telomerase, oncofetal proteins, e.g. alpha feto protein, and testis antigen, e.g. NY-ESO-1. For example, a tumour specific antigen is one of the group comprising or consisting of tumour antigens resulting from mutations, shared tumour antigens, differentiation antigens, antigens overexpressed in tumours, especially Nras, Hras, Kras which are indicative of a neoplastic state, tumour-specific antigens of the MAGE (including MAGE-B5, MAGE-B6, MAGE, MAGE-C2, MAGE-C3, MAGE-D), HAGE, SAGE, SSX-2, BAGE, TRAG-3, and GAGE families, including NY-ESO-1, LAGE, CAMEL, as well as MUC1, most preferably tumour-specific mutant Ras, e.g. Nras, Nras G12V, or Kras, KrasG12D and other common mutations.

Exemplary tumour antigens resulting from mutations are for lung carcinoma FIASNGVKLV (SEQ ID NO: 1), for melanoma YSVYFNLPADTIYTN(SEQ ID NO: 2), for chronic myeloid leukemia SSKALQRPV (SEQ ID NO: 3) or GFKQSSKAL (SEQ ID NO: 4) or ATGFKQSSKALQRPVAS (SEQ ID NO: 5) , for melanoma EDLTVKIGDFGLATEKSRWSGSHQFEQLS (SEQ ID NO: 6), for colorectal, gastric, and endometrial carcinoma FLIIWQNTM (SEQ ID NO: 7), for head and neck squamous cell carcinoma FPSDSWCYF (SEQ ID NO: 8), for melanoma SYLDSGIHF (SEQ ID NO: 9), for melanoma FSWAMDLDPKGA (SEQ ID NO: 10), for melanoma ACDPHSGHFV(SEQ ID NO: 11), for melanoma AVCPWTWLR (SEQ ID NO: 12), for colorectal carcinoma TLYQDDTLTLQAAG (SEQ ID NO: 13) or TLYQDDTLTLQAAG (SEQ ID NO: 14), for myeloid leukemia TMKQICKKEIRRLHQY (SEQ ID NO: 15), for melanoma KILDAVVAQK (SEQ ID NO: 16), for lung squamous CC especially ETVSEQSNV (SEQ ID NO: 17), for acute lymphoblastic leukemia RIAECILGM (SEQ ID NO: 18) or IGRIAECILGMNPSR (SEQ ID NO: 19) or IGRIAECILGMNPSR (SEQ ID NO: 20), for acute myelogenous leukemia YVDFREYEYY (SEQ ID NO: 21), for melanoma MIFEKHGFRRTTPP (SEQ ID NO: 22), for melanoma TLDWLLQTPK (SEQ ID NO: 23), for melanoma WRRAPAPGA (SEQ ID NO: 24) or PVTWRRAPA (SEQ ID NO: 25), for renal cell carcinoma, for melanoma and renal cell carcinoma SLFEGIDIYT (SEQ ID NO: 26), for bladder tumour AEPINIQTW (SEQ ID NO: 27), for melanoma FLEGNEVGKTY (SEQ ID NO: 28), for non-small cell lung carcinoma FLDEFMEGV (SEQ ID NO: 29), for melanoma EEKLIVVLF (SEQ ID NO: 30), for melanoma SELFRSGLDSY (SEQ ID NO: 31) or FRSGLDSYV (SEQ ID NO: 32), for melanoma EAFIQPITR (SEQ ID NO: 33), for melanoma RVIKNSIRLTL (SEQ ID NO: 34), for melanoma KINKNPKYK (SEQ ID NO: 35), lung squamous cell carcinoma QQITKTEV (SEQ ID NO: 36), colorectal carcinoma SLYKFSPFPL (SEQ ID NO: 37), for melanoma KELEGILLL (SEQ ID NO: 38), for head and neck squamous cell carcinoma VVPCEPPEV (SEQ ID NO: 39), for promyelocytic leukemia NSNHVASGAGEAAIETQSSSSEEIV (SEQ ID NO: 40), for melanoma LLLDDLLVSI (SEQ ID NO: 41), for melanoma PYYFAAELPPRNLPEP (SEQ ID NO: 42), for pancreatic adenocarcinoma VVVGAVGVG (SEQ ID NO: 43), for melanoma ILDTAGREEY (SEQ ID NO: 44), for melanoma RPHVPESAF (SEQ ID NO: 45), for melanoma KIFSEVTLK (SEQ ID NO: 46), for melanoma SHETVIIEL (SEQ ID NO: 47), for sarcoma QRPYGYDQ1M (SEQ ID NO: 48), for colorectal carcinoma RLSSCVPVA (SEQ ID NO: 49), for melanoma GELIGILNAAKVPAD (SEQ ID NO: 50).

Exemplary shared tumour antigens are:
AARAVFLAL (SEQ ID NO: 51), YRPRPRRY (SEQ ID NO: 52), YYWPRPRRY (SEQ ID NO: 53), VLPDVFIRC(V) (SEQ ID NO: 54), MLAVISCAV (SEQ ID NO: 55), RQKRILVNL (SEQ ID NO: 56), NYNNFYRFL (SEQ ID NO: 57), EYSKECLKEF (SEQ ID NO: 58), EYLSLSDKI (SEQ ID NO: 59), MLMAQEALAFL (SEQ ID NO: 60), SLLMWITQC (SEQ ID NO: 61), LAAQERRVPR (SEQ ID NO: 62), ELVRRILSR (SEQ ID NO: 63), APRGVRMAV (SEQ ID NO: 64), SLLMWITQCFLPVF (SEQ ID NO: 65), QGAMLAAQERRVPRAAEVPR (SEQ ID NO: 66), AADHRQLQLSISSCLQQL (SEQ ID NO:67), CLSRRPWKRSWSAGSCPGMPHL (SEQ ID NO: 68), CLSRRPWKRSWSAGSCPGMPHL (SEQ ID NO: 69), ILSRDAAPLPRPG (SEQ ID NO: 70), AGATGGRGPRGAGA (SEQ ID NO: 71), EADPTGHSY (SEQ ID NO: 72), KVLEYVIKV (SEQ ID NO: 73), SLFRAVITK (SEQ ID NO: 74), EVYDGREHSA (SEQ ID NO: 75), RVRFFFPSL (SEQ ID NO: 76), EADPTGHSY (SEQ ID NO: 77), REPVTKAEML (SEQ ID NO: 78), DPARYEFLW (SEQ ID NO: 79), ITKKVADLVGF (SEQ ID NO: 80), SAFPTTINF (SEQ ID NO: 81), SAYGEPRKL (SEQ ID NO: 82), SAYGEPRKL (SEQ ID NO: 83), TSCILESLFRAVITK (SEQ ID NO: 84), PRALAETSYVKVLEY (SEQ ID NO: 85), FLLLKYRAREPVTKAE (SEQ ID NO: 86), EYVIKVSARVRF (SEQ ID NO: 87), YLQLVFGIEV (SEQ ID NO: 88), EYLQLVFGI (SEQ ID NO: 89), REPVTKAEML (SEQ ID NO: 90), EGDCAPEEK (SEQ ID NO: 91), LLKYRAREPVTKAE (SEQ ID NO: 92), EVDPIGHLY (SEQ ID NO: 93), FLWGPRALV (SEQ ID NO: 94), KVAELVHFL (SEQ ID NO: 95), TFPDLESEF (SEQ ID NO: 96), VAELVHFLL (SEQ ID NO: 97), MEVDPIGHLY (SEQ ID NO: 98), EVDPIGHLY (SEQ ID NO: 99), REPVTKAEML (SEQ ID NO: 100), AELVHFLLL (SEQ ID NO: 101), MEVDPIGHLY (SEQ ID NO: 102), WQYFFPVIF (SEQ ID NO: 103), EGDCAPEEK (SEQ ID NO: 104), KKLLTQHFVQENYLEY (SEQ ID NO: 105), KKLLTQHFVQENYLEY (SEQ ID NO: 106), ACYEFLWGPRALVETS (SEQ ID NO: 107), VIFSKASSSLQL (SEQ ID NO: 108), VIFSKASSSLQL (SEQ ID NO: 109), GDNQIMPKAGLLIIV (SEQ ID NO: 110), TSYVKVLHHMVKISG (SEQ ID NO: 111), RKVAELVHFLLLKYRA (SEQ ID NO: 112), FLLLKYRAREPVTKAE (SEQ ID NO: 113), EVDPASNTY (SEQ ID NO: 114), GVYDGREHTV (SEQ ID NO: 115), NYKRCFPVI (SEQ ID NO: 116), SESLKMIF (SEQ ID NO: 117), MVKISGGPR (SEQ ID NO: 118), EVDPIGHVY (SEQ ID NO: 119), REPVTKAEML (SEQ ID NO: 120), EGDCAPEEK (SEQ ID NO: 121), ISGGPRISY (SEQ ID NO: 122), LLKYRAREPVTKAE (SEQ ID NO: 123), ALSVMGVYV (SEQ ID NO: 124), GLYDGMEHL (SEQ ID NO: 125), DPARYEFLW (SEQ ID NO: 126), FLWGPRALV (SEQ ID NO: 127), VRIGHLYIL (SEQ ID NO: 128), EGDCAPEEK (SEQ ID NO: 129), REPFTKAEMLGSVIR (SEQ ID NO: 130), AELVHFLLLKYRAR (SEQ ID NO: 131), LLFGLALIEV (SEQ ID NO: 132), ALKDVEERV (SEQ ID NO: 133), SESIKKKVL (SEQ ID NO: 134), PDTRPAPGSTAPPAHGVTSA (SEQ ID NO: 135), QGQHFLQKV (SEQ ID NO: 136), SLLMWITQC (SEQ ID NO: 137), MLMAQEALAFL (SEQ ID NO: 138), ASGPGGGAPR (SEQ ID NO: 139), LAAQERRVPR (SEQ ID NO: 140), TVSGNILTIR (SEQ ID NO: 141), APRGPHGGAASGL (SEQ ID NO: 142), MPFATPMEA (SEQ ID NO: 143), KEFTVSGNILTI (SEQ ID NO: 144), MPFATPMEA (SEQ ID NO: 145), LAMPFATPM (SEQ ID NO: 146), ARGPESRLL (SEQ ID NO: 147), SLLMWITQCFLPVF (SEQ ID NO: 148), LLEFYLAMPFATPMEAELARRSLAQ (SEQ ID NO: 149), LLEFYLAMPFATPMEAELARRSLAQ (SEQ ID NO: 150), EFYLAMPFATPM (SEQ ID NO: 151), RLLEFYLAMPFA (SEQ ID NO: 152), QGAMLAAQERRVPRAAEVPR (SEQ ID NO: 153), PGVLLKEFTVSGNILTIRLT (SEQ ID NO: 154), VLLKEFTVSG (SEQ ID NO: 155), AADHRQLQLSISSCLQQL (SEQ ID NO: 156), LLEFYLAMPFATPMEAELARRSLAQ (SEQ ID NO: 157), LKEFTVSGNILTIRL (SEQ ID NO:158 ), PGVLLKEFTVSGNILTIRLTAADHR (SEQ ID NO: 159), LLEFYLAMPFATPMEAELARRSLAQ (SEQ ID NO: 160), AGATGGRGPRGAGA (SEQ ID NO: 161), LYATVIHDI (SEQ ID NO: 162), ILDSSEEDK (SEQ ID NO: 163), KASEKIFYV (SEQ ID NO: 164), EKIQKAFDDIAKYFSK (SEQ ID NO: 165), WEKMKASEKIFYVYMKRK (SEQ ID NO: 166), KIFYVYMKRKYEAMT (SEQ ID NO: 167), KIFYVYMKRKYEAM (SEQ ID NO: 168), INKTSGPKRGKHAWTHRLRE (SEQ ID NO: 169), YFSKKEWEKMKSSEKIVYVY (SEQ ID NO: 170), MKLNYEVMTKLGFKVTLPPF (SEQ ID NO: 171), KHAWTHRLRERKQLVVYEEI (SEQ ID NO: 172), LGFKVTLPPFMRSKRAADFH (SEQ ID NO: 173), KSSEKIVYVYMKLNYEVMTK (SEQ ID NO: 174), KHAWTHRLRERKQLVVYEEI (SEQ ID NO: 175), SLGWLFLLL (SEQ ID NO: 176), LSRLSNRLL (SEQ ID NO: 177), LSRLSNRLL (SEQ ID NO: 178), CEFHACWPAFTVLGE (SEQ ID NO: 179), CEFHACWPAFTVLGE (SEQ ID NO: 180), CEFHACWPAFTVLGE (SEQ ID NO: 181), EVISCKLIKR (SEQ ID NO: 182) or CATWKVICKSCISQTPG (SEQ ID NO: 183).

Exemplary tumour differentiation antigens are:
YLSGANLNL (SEQ ID NO: 184), IMIGVLVGV (SEQ ID NO: 185), GVLVGVALI (SEQ ID NO: 186), HLFGYSWYK (SEQ ID NO: 187), QYSWFVNGTF (SEQ ID NO: 188), TYACFVSNL (SEQ ID NO: 189), AYVCGIQNSVSANRS (SEQ ID NO: 190), DTGFYTLHVIKSDLVNEEATGQFRV (SEQ ID NO: 191), YSWRINGIPQQHTQV (SEQ ID NO: 192), TYYRPGVNLSLSC (SEQ ID NO: 193), EIIYPNASLLIQN (SEQ ID NO: 194), YACFVSNLATGRNNS (SEQ ID NO: 195), LWWVNNQSLPVSP (SEQ ID NO: 196), LWWVNNQSLPVSP (SEQ ID NO: 197), LWWVNNQSLPVSP (SEQ ID NO: 198), EIIYPNASLLIQN (SEQ ID NO: 199), NSIVKSITVSASG (SEQ ID NO: 200), KTWGQYWQV (SEQ ID NO: 201), (A)MLGTHTMEV (SEQ ID NO: 202), ITDQVPFSV (SEQ ID NO: 203), YLEPGPVTA (SEQ ID NO: 204), LLDGTATLRL (SEQ ID NO: 205), VLYRYGSFSV (SEQ ID NO: 206), SLADTNSLAV (SEQ ID NO: 207), RLMKQDFSV (SEQ ID NO: 208), RLPRIFCSC (SEQ ID NO: 209), LIYRRRLMK (SEQ ID NO: 210), ALLAVGATK (SEQ ID NO: 211), IALNFPGSQK (SEQ ID NO: 212), ALNFPGSQK (SEQ ID NO: 213), ALNFPGSQK (SEQ ID NO: 214), VYFFLPDHL (SEQ ID NO: 215), RTKQLYPEW (SEQ ID NO: 216), HTMEVTVYHR (SEQ ID NO: 217), SSPGCQPPA (SEQ ID NO: 218), VPLDCVLYRY (SEQ ID NO: 219), LPHSSSHWL (SEQ ID NO: 220), SNDGPTLI (SEQ ID NO: 221), GRAMLGTHTMEVTVY (SEQ ID NO: 222), WNRQLYPEWTEAQRLD (SEQ ID NO: 223), TTEWVETTARELPIPEPE (SEQ ID NO: 224), TGRAMLGTHTMEVTVYH (SEQ ID NO: 225), GRAMLGTHTMEVTVY (SEQ ID NO: 226), SVSESDTIRSISIAS (SEQ ID NO: 227), LLANGRMPTVLQCVN (SEQ ID NO: 228), RMPTVLQCVNVSVVS (SEQ ID NO: 229), PLLENVISK (SEQ ID NO: 230), (E)AAGIGILTV (SEQ ID NO: 231), ILTVILGVL (SEQ ID NO: 232), EAAGIGILTV (SEQ ID NO: 234), AEEAAGIGIL(T) (SEQ ID NO: 235), RNGYRALMDKS (SEQ ID NO: 236), EEAAGIGILTVI (SEQ ID NO: 237), AAGIGILTVILGVL (SEQ ID NO: 238), APPAYEKLpSAEQ (SEQ ID NO: 239), EEAAGIGILTVI (SEQ ID NO: 240), RNGYRALMDKSLHVGTQCALTRR (SEQ ID NO: 241), MPREDAHFIYGYPKKGHGHS (SEQ ID NO: 242), KNCEPVVPNAPPAYEKLSAE (SEQ ID NO: 243), SLSKILDTV (SEQ ID NO: 244), LYSACFWWL (SEQ ID NO: 245), FLTPKKLQCV (SEQ ID NO: 246), VISNDVCAQV (SEQ ID NO: 247), VLHWDPETV (SEQ ID NO: 248), MSLQRQFLR (SEQ ID NO: 249), ISPNSVFSQWRVVCDSLEDYD (SEQ ID NO: 250), SLPYWNFATG (SEQ ID NO: 251), SVYDFFVWL (SEQ ID NO: 252), TLDSQVMSL (SEQ ID NO: 253), LLGPGRPYR (SEQ ID NO: 254), LLGPGRPYR (SEQ ID NO: 255), ANDPIFVVL (SEQ ID NO: 256), QCTEVRADTRPWSGP (SEQ ID NO: 257), ALPYWNFATG (SEQ ID NO: 258), KCDICTDEY (SEQ ID NO: 259), SSDYVIPIGTY (SEQ ID NO: 260), MLLAVLYCL (SEQ ID NO: 261), CLLWSFQTSA (SEQ ID NO: 262), YMDGTMSQV (SEQ ID NO: 263), AFLPWHRLF (SEQ ID NO: 264), QCSGNFMGF (SEQ ID NO: 265), TPRLPSSADVEF (SEQ ID NO: 266), LPSSADVEF (SEQ ID NO: 267), LHHAFVDSIF (SEQ ID NO: 268), SEIWRDIDF (SEQ ID NO: 269), QNILLSNAPLGPQFP (SEQ ID NO: 270), SYLQDSDPDSFQD (SEQ ID NO: 271) or FLLHHAFVDSIFEQWLQRHRP (SEQ ID NO: 272).

Exemplary antigens overexpressed in tumour are:
SVASTITGV (SEQ ID NO: 273), RSDSGQQARY (SEQ ID NO: 274), LLYKLADLI (SEQ ID NO: 275), YLNDHLEPWI (SEQ ID NO: 276), CQWGRLWQL (SEQ ID NO: 277), VLLQAGSLHA (SEQ ID NO: 278), KVHPVIWSL (SEQ ID NO: 279), LMLQNALTTM (SEQ ID NO: 280), LLGATCMFV (SEQ ID NO: 281), NPPSMVAAGSVVAAV (SEQ ID NO: 282), ALGGHPLLGV (SEQ ID NO: 283), TMNGSKSPV (SEQ ID NO: 284), RYQLDPKFI (SEQ ID NO: 285), DVTFNIICKKCG (SEQ ID NO: 286), FMVEDETVL (SEQ ID NO: 287), FINDEIFVEL (SEQ ID NO: 288), KYDCFLHPF (SEQ ID NO: 289), KYVGIEREM (SEQ ID NO: 290), NTYASPRFK (SEQ ID NO: 291), HLSTAFARV (SEQ ID NO: 292), KIFGSLAFL (SEQ ID NO: 293), IISAWGIL (SEQ ID NO: 294), ALCRWGLLL (SEQ ID NO: 295), ILHNGAYSL (SEQ ID NO: 296), RLLQETELV (SEQ ID NO: 297), VVLGVVFGI (SEQ ID NO: 298), YMIMVKCWMI (SEQ ID NO: 299), HLYQGCQVV (SEQ ID NO: 300), YLVPQQGFFC(SEQ ID NO: 301), PLQPEQLQV (SEQ ID NO: 302), TLEEITGYL (SEQ ID NO: 303), ALIHHNTHL (SEQ ID NO: 304), PLTSIISAV (SEQ ID NO: 305), VLRENTSPK (SEQ ID NO: 306), TYLPTNASL (SEQ ID NO: 307), ALLEIASCL (SEQ ID NO: 308), WLPFGFILI (SEQ ID NO: 309), SPRWWPTCL (SEQ ID NO: 310), GVALQTMKQ (SEQ ID NO: 311), FMNKFIYEI (SEQ ID NO: 312), QLAVSVILRV (SEQ ID NO: 313), LPAVVGLSPGEQEY (SEQ ID NO: 314), VGQDVSVLFRVTGALQ (SEQ ID NO: 315), VLFYLGQY (SEQ ID NO: 316), TLNDECWPA (SEQ ID NO: 317), GLPPDVQRV (SEQ ID NO: 318), SLFPNSPKWTSK (SEQ ID NO: 319), STAPPVHNV (SEQ ID NO: 320), LLLLTVLTV (SEQ ID NO: 321), PGSTAPPAHGVT (SEQ ID NO: 322), LLGRNSFEV (SEQ ID NO: 323), RMPEAAPPV (SEQ ID NO: 324), SQKTYQGSY (SEQ ID NO: 325), PGTRVRAMAIYKQ (SEQ ID NO: 326), HLIRVEGNLRVE (SEQ ID NO: 327), TLPGYPPHV (SEQ ID NO: 328), CTACRWKKACQR (SEQ ID NO: 329), VLDGLDVLL (SEQ ID NO: 330), SLYSFPEPEA (SEQ ID NO: 331), ALYVDSLFFL (SEQ ID NO: 332), SLLQHLIGL (SEQ ID NO: 333), LYVDSLFFL (SEQ ID NO: 334), NYARTEDFF (SEQ ID NO: 335), LKLSGVVRL (SEQ ID NO: 336), PLPPARNGGL (SEQ ID NO: 337), SPSSNRIRNT (SEQ ID NO: 338), LAALPHSCL (SEQ ID NO: 339), GLASFKSFLK(SEQ ID NO: 340), RAGLQVRKNK (SEQ ID NO: 341), ALWPWLLMA(T) (SEQ ID NO: 342), NSQPVWLCL (SEQ ID NO: 343), LPRWPPPQL (SEQ ID NO: 344), KMDAEHPEL (SEQ ID NO: 345), AWISKPPGV (SEQ ID NO: 346), SAWISKPPGV (SEQ ID NO: 347), MIAVFLPIV (SEQ ID NO: 348), HQQYFYKIPILVINK (SEQ ID NO: 349), ELTLGEFLKL (SEQ ID NO: 350), ILAKFLHWL (SEQ ID NO: 351), RLVDDFLLV (SEQ ID NO: 352), RPGLLGASVLGLDDI (SEQ ID NO: 353), LTDLQPYMRQFVAHL (SEQ ID NO: 354), SRFGGAVVR (SEQ ID NO: 355), TSEKRPFMCAY (SEQ ID NO: 356), CMTWNQMNL (SEQ ID NO: 357), LSHLQMHSRKH (SEQ ID NO: 358) or KRYFKLSHLQMHSRKH (SEQ ID NO: 359)

A preferred tumour antigen is a lysate or homogenate of the tumour to be treated, more preferably a fraction thereof soluble in aqueous media, e.g. soluble in physiological saline and/or in medium containing DCs. Tumour lysate or tumour homogenate can e.g. originate from a tumour biopsy or from a surgery of the later recipient of the pharmaceutical combination of compounds.

It has been found that the administration of the first composition and subsequent administration of the second composition results in the generation of target antigen-specific activated T-cells, which preferably are CD8+ T-cells and/or CD4+ T-cells. In comparison to other compositions, the combination of the invention results also in a higher proportion of target antigen-specific activated T-cells of all activated T-cells. The number and proportion of target antigen-specific T-cells was determined by staining a peripheral blood sample for IFN gamma (IFNγ) following in vitro contacting with the target antigen using brefeldin A (GolgiPlug, available from Becton Dickinson) and immune staining with a labelled anti-IFN gamma antibody and detection in flow cytometry. The number of all activated T-cells was determined by staining a peripheral blood sample with anti-CD11a antibody and detection in flow cytometry. Accordingly, activated CD8+ T-cells are CD11a^{hi}, tetramer-positive for the antigen and/or are IFNγ positive. The effect of inducing a target antigen-specific T-cell response, wherein the target antigen preferably is a tumour antigen, within e.g. 10 to 14 days is currently believed to be based on the combination of the first composition providing the target antigen in cationic liposomes which specifically prime DC in the recipient for the target antigen, with the subsequent administration of the second composition providing a specific boost for the T-cells that were stimulated by the DCs primed with target antigen by the combination of the target antigen with the co-stimulatory antibody for T-cells, preferably in combination with an adjuvant, e.g. a non-specific agonist for TLR3, for TLR7, for TLR4 and/or for TLR9, a NOD like receptor agonist, a RIG-I like receptor agonist, a STING pathway agonist, and mixtures of at least two of these. Accordingly, the first composition and its administration can also be termed priming, and the second composition and its administration can be termed boosting.

It is currently assumed that the second composition due to its content of the target antigen preferentially boosts target antigen-specific T-cells from the pool of primed T-cells present in the recipient. This is an advantage over the use of co-stimulating antibody and/or of a non-specific TLR3 agonist alone or in combination as these can be expected to activate all primed T-cells irrespective of their antigen specificity, resulting in a proportionate boost of target antigen-specific T-cells only. Accordingly, the second composition is also assumed to have the advantage of boosting non-target specific T-cells to a lesser extent than the use of co-stimulating antibody and/or of a non-specific TLR3 agonist alone. Optionally, the combination of the first composition and of the second composition, which is for administration subsequent to administration of the first composition, is for administration of each of these compositions only once. Generally optionally, the second composition is for administration one time only in the treatment of one patient. Optional and preferred the administration of the second composition is limited to exactly one administration.

Advantageously, the administration of the second composition following the administration of the first composition is with a temporal delay of approx. at least 2 or at least 3 to at least 7 days, e.g. of 3 to 5 or to 10 days.

The pharmaceutical combination of compounds has the advantage of raising in a recipient an effective T-cell immunity also against a target antigen which is an intracellular tumour antigen.

For the purpose of the invention, an antibody, e.g. a co-stimulatory agonistic antibody for CD8+ T-cells, can be a natural antibody, e.g. IgG, or a synthetic peptide having a paratope of the specificity, e.g. a diabody, minibody etc., or a physiological ligand for the co-stimulatory receptor.

The invention is now described in greater detail by way of mouse experiments with reference to the figures, which show for different first and second compositions administered to experimental animals in
- Fig. 1 flow cytometry results of peripheral blood with pre-gating for CD90.2/CD8 with staining for CD8 and IFNγ at day -1 prior to administration of the second composition at a), c), e), g), i), k) and m) without ex vivo restimulation with the target antigen (Adpgk), and at b), d), f), h), j),l) and n) with ex vivo restimulation with the antigen Adpgk,

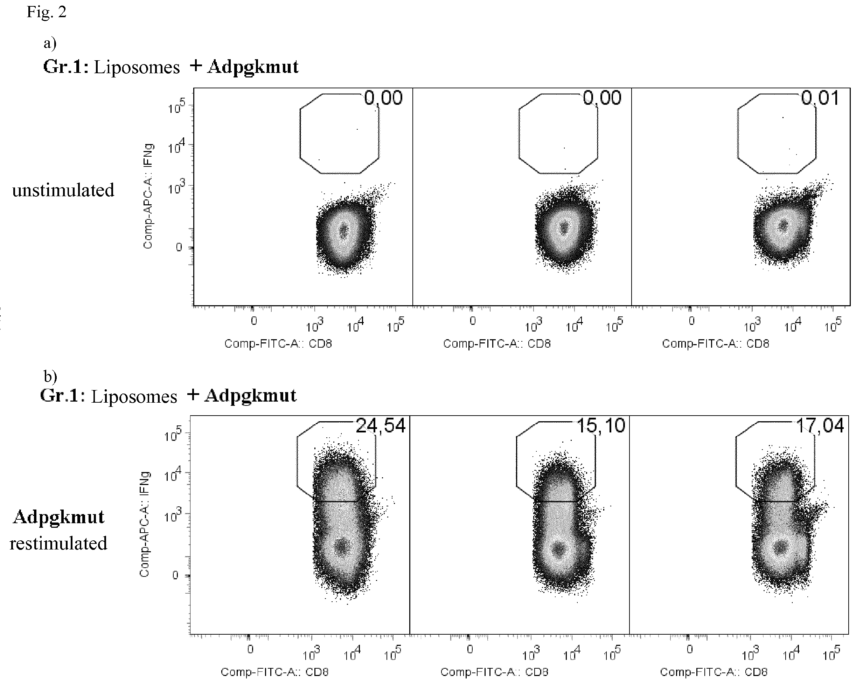

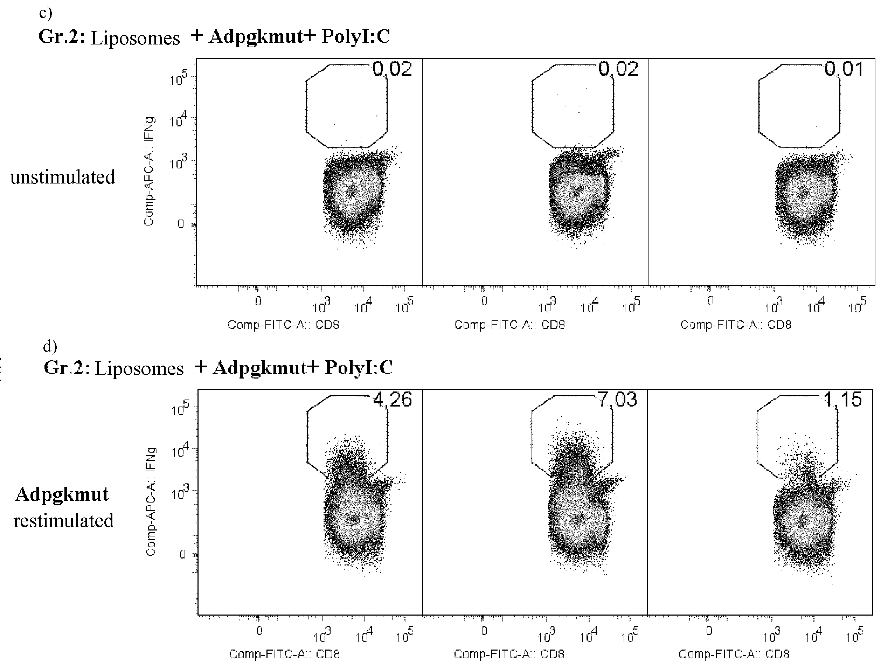

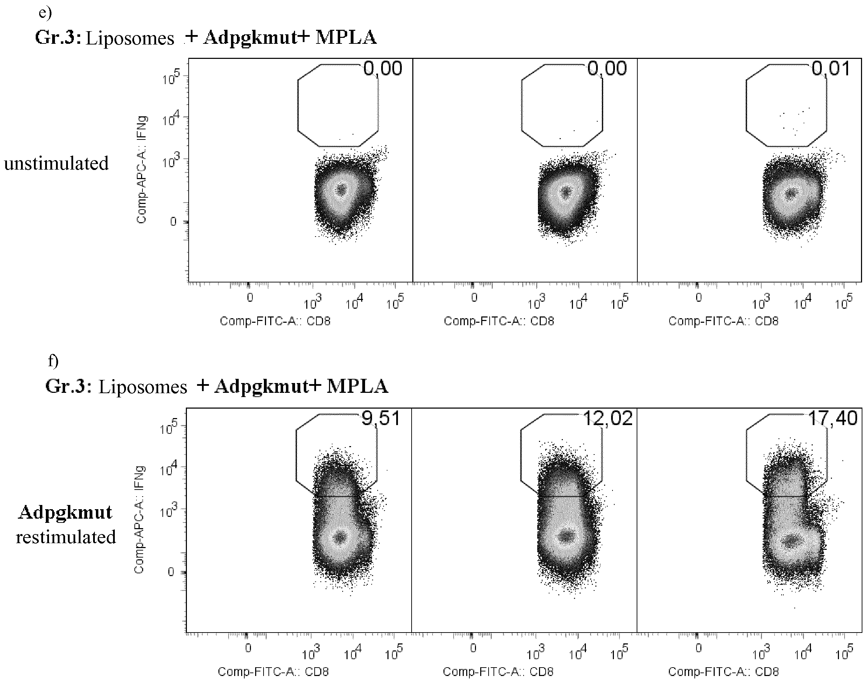

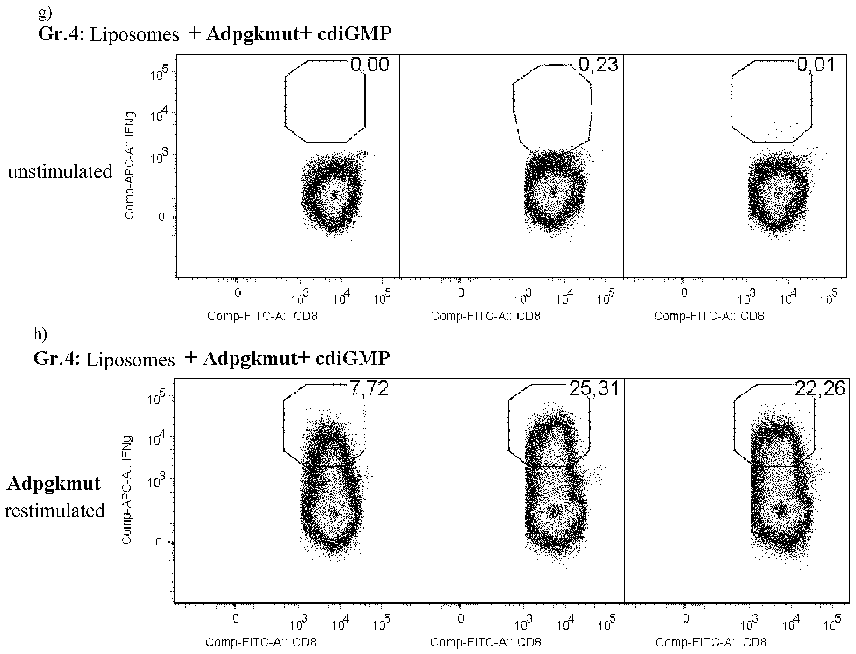

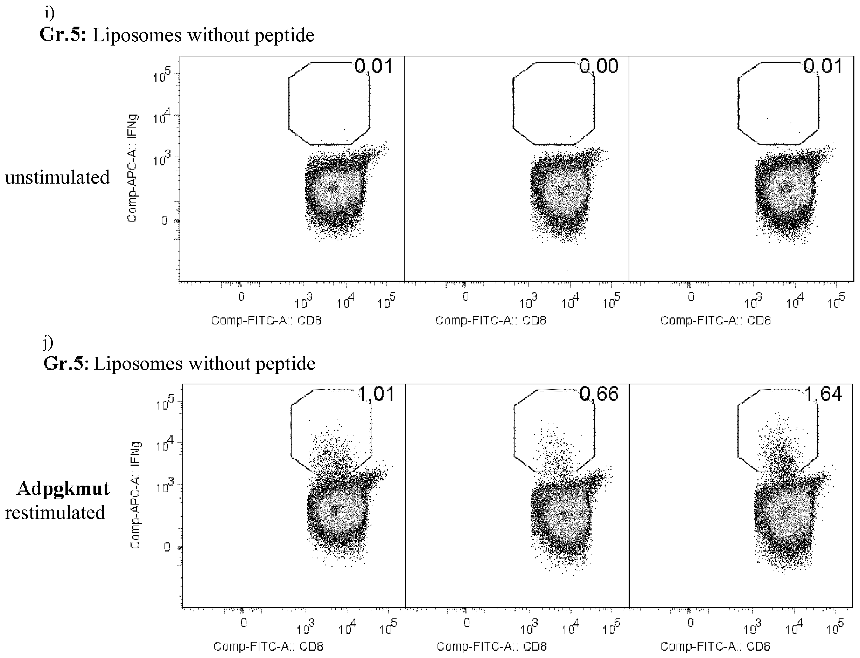

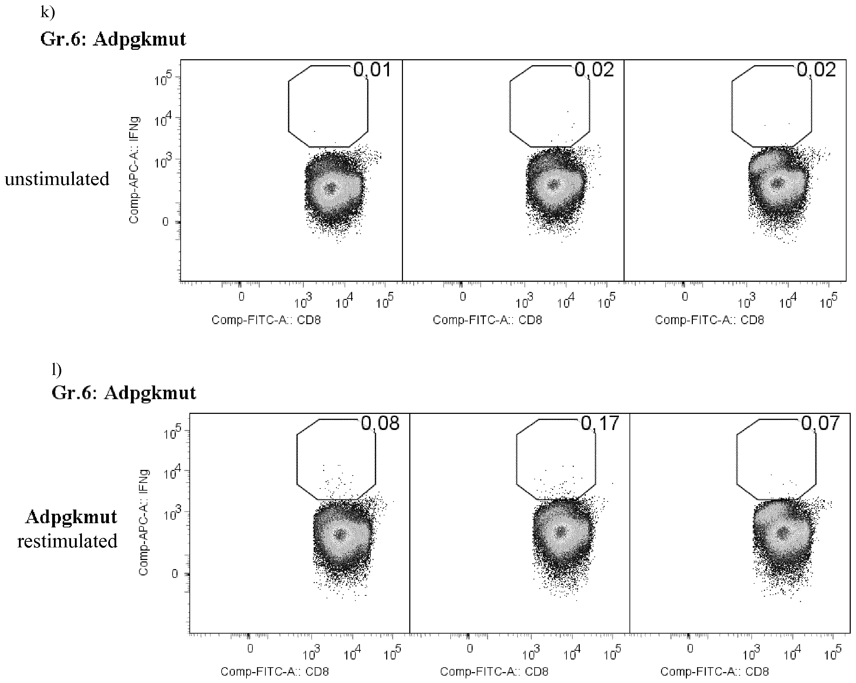

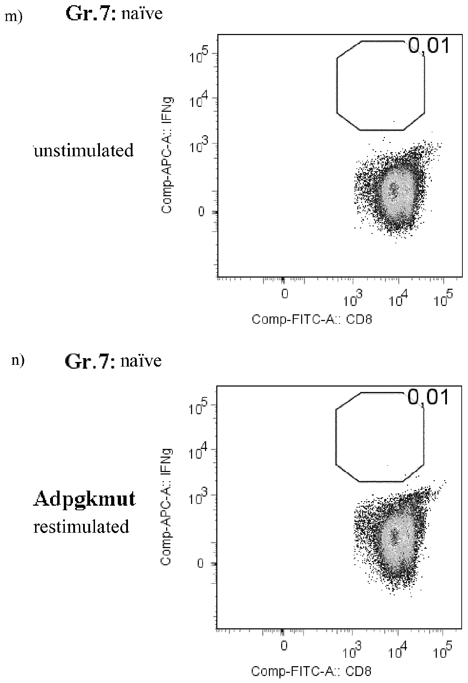

- Fig. 2 flow cytometry results of peripheral blood with pre-gating for CD90.2/CD8 with staining for CD8 and IFNγ at day 7 following administration of the second composition at a), c), e), g), i), k) and m) without ex vivo restimulation with the target antigen (Adpgk), and at b), d), f), h), j),l) and n) with ex vivo restimulation with the antigen Adpgk,
- Fig. 3 flow cytometry results with staining for CD4+ and for IFNγ of peripheral blood of mice immunized with liposomes containing the CD4 epitope MTAG85B and c-di-GMP on day -7 and boosted with anti-CD antibody, Poly I:C and soluble MTAG85B on day 0,
- Fig. 4 a) to d) flow cytometry results with staining for IFNγ and CD8+ for peripheral blood of a comparative example (PLGA) and of an example according to the invention, and
- Fig. 5 a dot plot for the results of the comparative example and of the example according to the invention.

In the following examples and comparative examples, mice were used for representing a human recipient. Mice were divided into groups of 3 mice (strain C57 Bl/6) each. The animals were housed under standard conditions with feed and water ad libitum. Mice were subjected to different prime-boost regimens. Administration of first composition (priming) and of second composition (boosting) was by intravenous (iv) injection. In the figures, the co-stimulatory antibody is designated by its target, e.g. in the figures anti-CD40 antibody is designated as CD40ab.

As an example for a malignant target antigen, the neoantigen Adpgk (herein also referred to as Adpgkmut) having amino acid sequence ASMTNMELM (SEQ ID NO: 360) was used. Adpgk is a model antigen for a homologous tumour antigen. Adpgk was prepared by chemical peptide synthesis. The compositions comprised the constituents of the compositions in aqueous medium, preferably in physiological saline.

### Example 1: Immunization with different first compositions, followed by different second compositions

Cationic liposomes were prepared by dissolving 1,2-distearoyl-sn-glycero-3-phosphocholine (DSPC), 1,2-dipalmitoyl-3-trimethylammonium-propane (DPTAP) and cholesterol in a molar ratio of 4:1:2 in dichloromethane and cast onto a glass plate to obtain 10 mg of a lipid film after evaporation of the dichloromethane. The lipid film was rehydrated with an aqueous solution containing 0.5 mg of the model antigen Adpgk. The rehydrated lipid composition is lyophilised for removal of solvent water and rehydrated with 10 mM phosphate buffer, pH 7.4, to obtain multilamellar vesicles. These vesicles were extruded through a membrane having pores of 200 nm to obtain antigen-containing monodisperse unilamellar cationic liposomes which have a diameter of 160 nm.

For priming, on day -7 mice received as a first composition in
Group 1 20 µg liposomes containing Adpgk,
Group 2 20 µg liposomes containing Adpgk in admixture with Poly(I:C),
Group 3 20 µg liposomes containing Adpgk in admixture with monophosphoryl lipid A (MPLA),
Group 4 20 µg liposmes containing Adpgk in admixture with cyclic diguanosine monophosphate (cdiGMP),
Group 5 20 µg liposomes containing no added antigen,
Group 6 20 µg of the antigen Adpgk only, and
Group 7 saline (naive) only, intravenously.

In these liposomes, the TLR4 agonist MPLA was used as an exemplary TLR agonist and cdiGMP was used as an exemplary STING pathway agonist.

7 days later (day 0), mice received boosting by intravenous administration of a combination of 100µg Adpgk peptide, 100µg of agonistic anti-CD40 antibody (clone 1C10) and 200µg of Poly(I:C) as the second composition. After the administration of the second composition, mice were bled from the mandibular vein on day 7. After red cell lysis, peripheral blood mononuclear cells were stained with the following labelled antibodies: anti-IFN gamma antibody-APC (clone XMG1.2, eBioscience), anti-CD8 antibody-FITC (53-6.7, eBioscience and Becton Dickinson Biosciences).

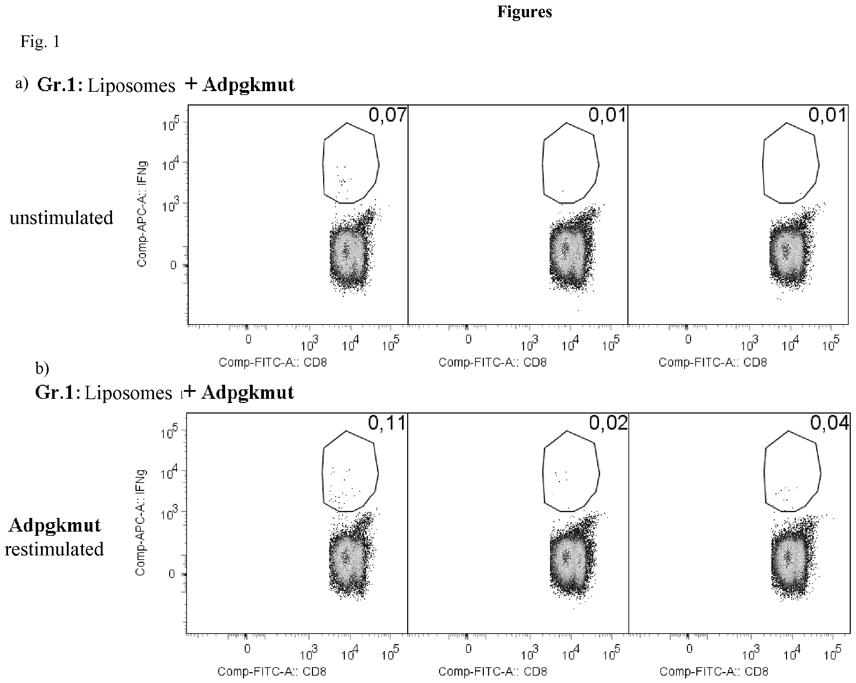

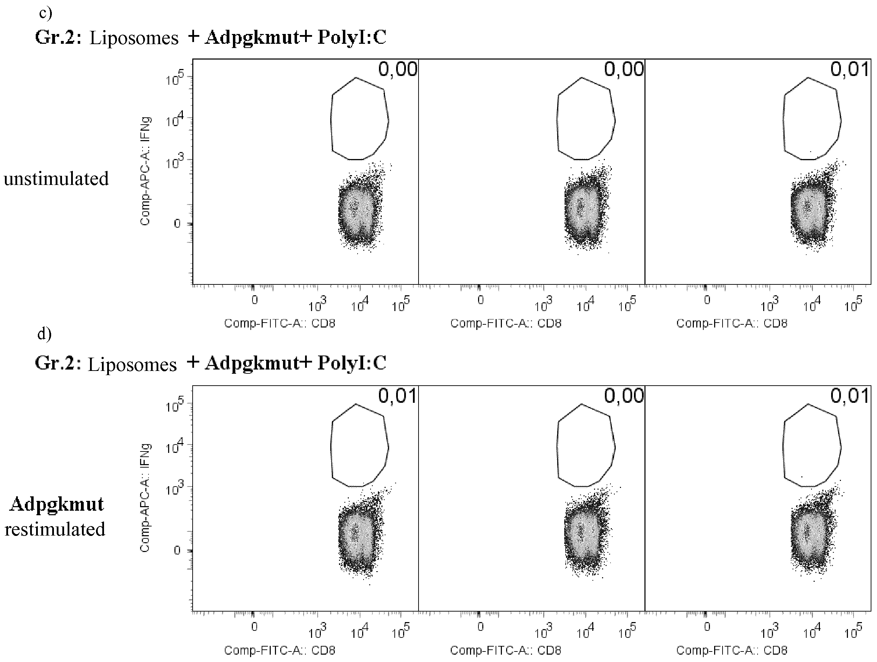

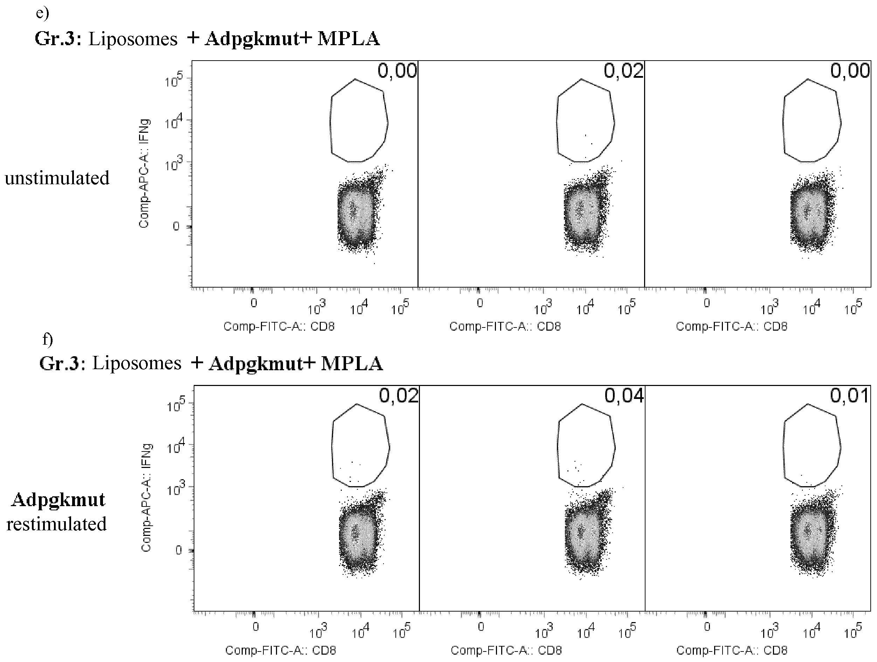

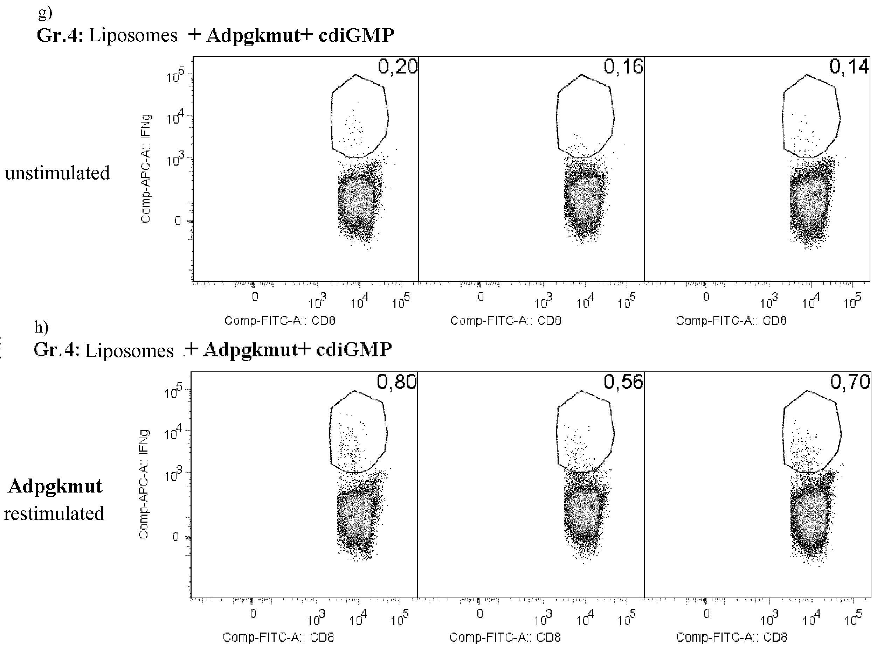

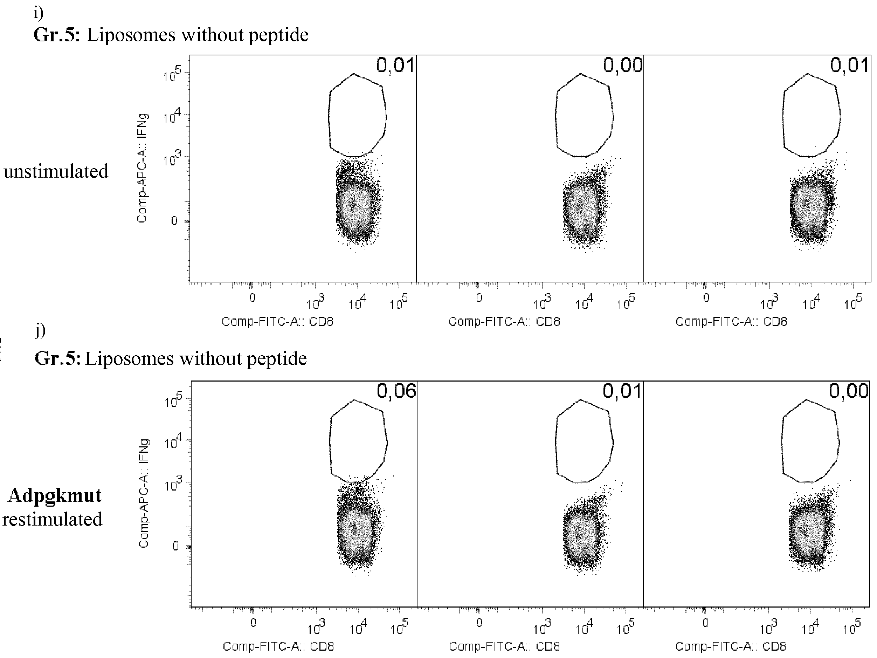

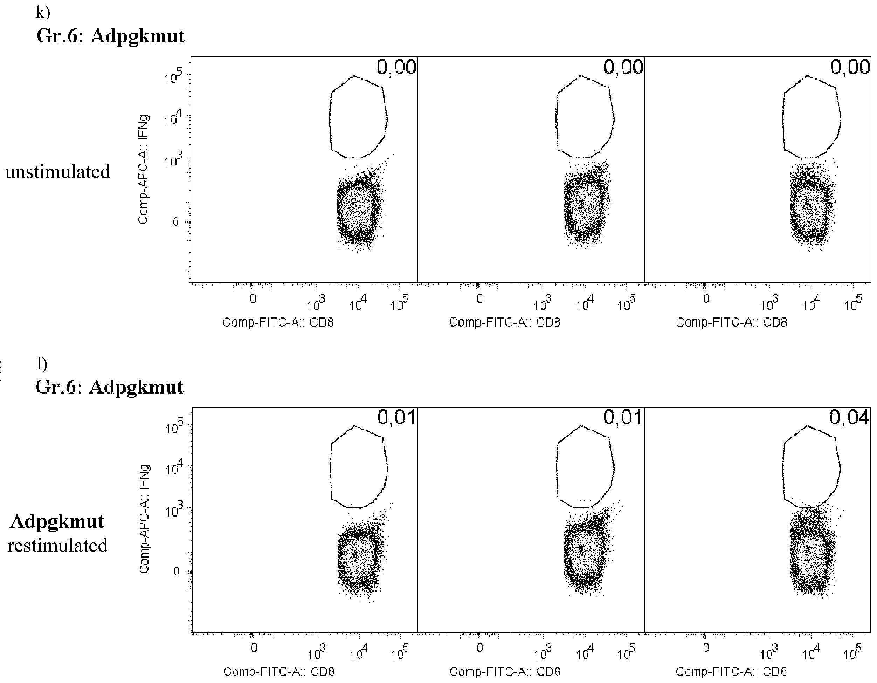

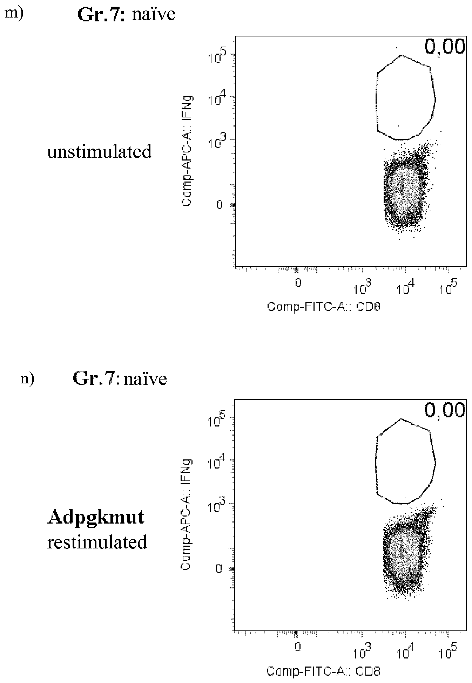

The results of flow cytometry (FACS) using a model Canto II flow cytometer (Becton Dickinson Biosciences) are shown in Fig. 1 for each of the 3 mice at day -1, i.e. 6 days after the administration of the first composition and 1 day prior to administration of the second composition.

The 3 animals of each group were treated identically.

Figure 1 shows the FACS results of analysis for IFNγ (Comp-APC-A:IFNg), indicating the activated T-cells of the total T-cells (CD8, Comp-FITC-A:CD8), without ex vivo restimulation by the antigen (unstimulated) at a), c), e), g), i), k) and m) in one sample of each animal and with added antigen Adpgk (Adpgkmut) for each sample of each animal (Figs. b), d), f), h), j), 1), m) and n).

The analysis for target antigen-specific T-cells was by measuring IFNγ following restimulation with antigen Adpgk in a sample of each animal in comparison to the samples from the same animals without added antigen. For measurement of IFNγ produced by each T-cell (CD8), secretion of IFNγ was hindered by addition of brefeldin A (GolgiPlug, available from Becton Dickinson), followed by staining using a labelled anti-IFNy antibody and measurement by flow cytometry.

These results show that the combination of the first composition and the second composition, which are for subsequent administration, generates an effective immune response. This shows that the synthetic compositions, which are devoid of cells, are suitable for eliciting an immune response by antigen specific CD8+ T-cells.

Generally, the first composition can optionally be free from a co-stimulatory antibody and/or free from a TLR agonist, or a TLR agonist and/or a STING agonist can be incorporated within the liposomes of the first composition.

### Example 2: Immunization against a self-antigen

Mice were immunized with a first composition of liposomes containing the CD4 epitope MTAG85B as a model homologous tumour antigen, and cdi-GMP (cyclic di-guanylate) on day -7. On day 0, the animals as a boost received a second composition consisting of anti-CD40 antibody, Poly I:C and soluble MTAG85B as the antigen (MTAG85B).

For comparison, a group of mice received the same first composition but no second composition (unstimulated).

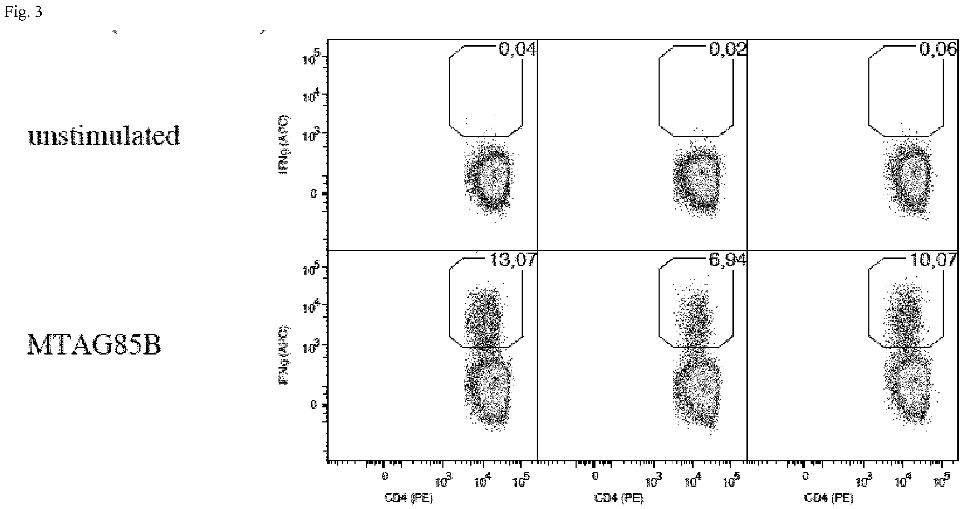

For analysis, FACS of peripheral blood taken on day 7 was performed with staining for IFNγ and for CD4 (anti-CD4 antibody conjugated to phycoerythritin (CD4(PE)), detecting CD4+ T-cells which are activated (high IFNγ). The results are depicted in Fig. 3 and show intracellular cytokine staining for IFNγ on day 7 on CD4-positive T cells following administration of the composition according to the invention (MTAG85B), whereas hardly any IFNγ-positive CD4+ T-cell was detected in the comparative mice (unstimulated).

This result shows that the pharmaceutical combination of compositions of the invention effectively also raises an immune response of CD4+ T-cells, whereas administration of only the first composition does not give a detectable CD4+ immune response.

### Example 3: Immunization against a peptide and comparative example

Using Adpgk as the antigen, 3 mice in each group were immunized with 100µg Adpgk peptide immobilized on PLGA microspheres for priming as described in Pham et al. (loc. cit.) for comparison, followed 7 days later by no boost or by administration of a second composition of 100µg Adpgk peptide, 100µg of agonistic anti-CD40 antibody (clone 1C10) and 200µg of cdi-GMP.

Additional groups of 3 mice each were immunized with cationic liposomes as described in Example 1, followed 7 days later by no boost as a further comparison, or followed by administration of a second composition of 100µg Adpgk peptide, 100µg of agonistic anti-CD40 antibody (clone 1C10) and 200µg of cdiGMP according to the invention.

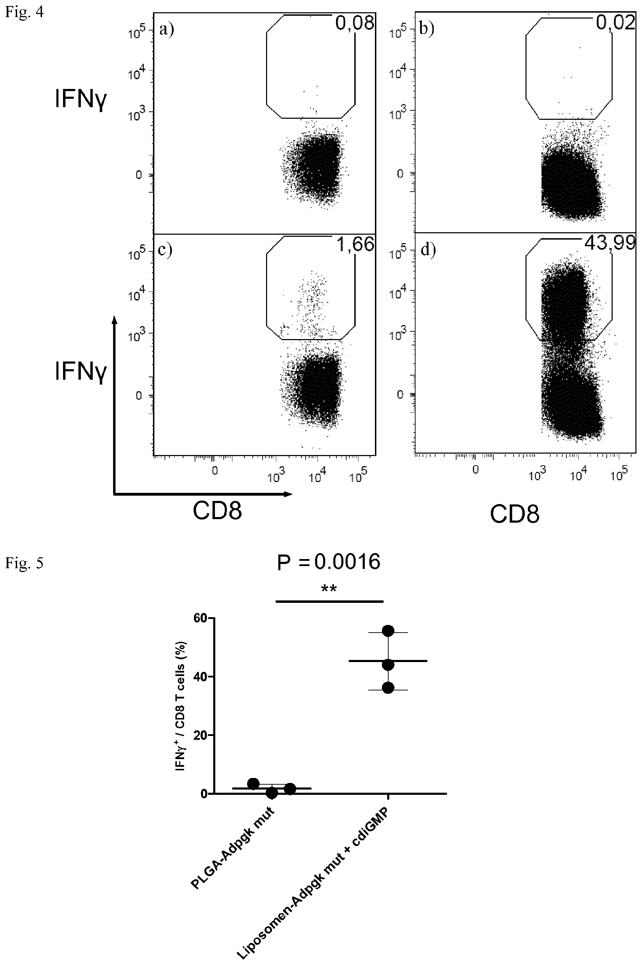

Fig. 4 shows the FACS results obtained by staining for CD8+ T-cells using anti-CD8 antibody-FITC antibody (CD8) and staining for anti-IFN gamma antibody-APC (IFNγ). Fig. 4a) shows the FACS result for the comparative example using the antigen (Adpgk mut) on PLGA microspheres (PLGA) only, i.e. without boost, Fig. 4b) shows the FACS results for the comparative example using the antigen on PLGA microspheres (PLGA) with subsequent boost using the second composition according to the invention, Fig. 4c) shows the FACS result obtained with administration of the first composition only, i.e. without the boost by the second composition, and 4d) shows the FACS result for the immunization using the combination of compounds according to the invention.

Fig. 5 summarizes the FACS results for the comparative example of administration of the antigen on PLGA microspheres followed by the second composition as used in the invention and results for the administration of the combination of compounds according to the invention (Liposomen-Adpgk mut + cdiGMP). Fig. 5 shows that the combination of the first composition and the second composition according to the invention results in an effective immune response, represented by a high proportion of activated, e.g. IFNγ-positive, CD8+ T-cells.

This result shows that using PLGA microspheres coated with a tumour antigen that were prepared according to Pham et al. as a replacement for the liposomes in the pharmaceutical composition for comparison, it was found that the pharmaceutical combination containing liposomes as the first composition according to the invention elicited a more intense immune response. This comparison demonstrates that administration of a first composition of the model peptide antigen on PLGA spheres according to Pham et al. followed by administration of the same second composition as in the invention only resulted in a weak immune response, and administration of the model peptide antigen on PLGA spheres according to Pham et al. only, i.e. without a boost by a second composition, resulted in almost no detectable CD8+ immune response.

## Claims

1. Pharmaceutical combination of compositions for use in medical treatment, the combination comprising a first composition comprising liposomes which contain a target antigen, and for administration at least 2 days subsequent to administration of the first composition, a second composition comprising at least a portion of the target antigen and a co-stimulatory antibody effective for activating T-cells and/or the dendritic cells (DC) which co-stimulatory antibody is selected from the group consisting of an anti-CD137 antibody, an anti-CD40 antibody, an anti-OX40 antibody, an anti-ICOS antibody, an anti-CD27 antibody, an anti-CD28 antibody, an anti-GITR antibody, specifically anti-human GITR/AITR antibody, an anti-HVEM antibody, an anti-TIM1 antibody, an anti-TIM3 antibody and mixtures of at least two of these, and wherein the target antigens of the first composition and of the second composition contain at least one identical epitope for MHC I and/or for MHC II.

2. Pharmaceutical combination of compositions for use in medical treatment according to claim 1, wherein the liposomes of the first composition are cationic liposomes.

3. Pharmaceutical combination of compositions for use in medical treatment according to one of the preceding claims, wherein the liposomes contain a compound effective for activating dendritic cells (DC) which is an adjuvant selected from the group comprising or consisting of Toll like receptor (TLR) agonists, NOD like receptor agonists, RIG-I like receptor agonists and STING pathway agonists, and mixtures of at least two of these.

4. Pharmaceutical combination of compositions for use in medical treatment according to claim 3, wherein the TLR agonist is selected from the group consisting of TLR1/2 agonists, e.g. lipoproteins and/or peptidoglycans, TLR3 agonists, e.g. double-stranded RNA, Poly(I:C) or PolyICLC, TLR4 agonists, e.g. lipopolysaccharides, TLR5 agonists, e.g. flagellin, TLR6 agonists, e.g. diacetylated lipoproteins, TLR7 agonists and TLR8 agonists, e.g. single-stranded RNA, TLR9 agonists, e.g. non-methylated GpG-containing DNA, TLR11 agonists, e.g. profilin, and combinations of at least two of these.

5. Pharmaceutical combination of compositions for use in medical treatment according to one of claims 2 to 4, wherein the compound effective for activating dendritic cells (DC) and/or the TLR agonist is enclosed within the liposomes only.

6. Pharmaceutical combination of compositions for use in medical treatment according to one of the preceding claims, wherein at least one of the compounds of the second composition is formulated in liposomes, and the co-stimulatory antibody effective for activating dendritic cells (DC) and/or T-cells and/or the adjuvant is arranged on the outside of the liposomes and/or within the liposomes only.

7. Pharmaceutical combination of compositions for use in medical treatment according to one of the preceding claims, wherein the medical treatment is the treatment of tumour, of viral infections or of infections by intracellular bacteria.

8. Pharmaceutical combination of compositions for use in medical treatment according to one of the preceding claims, wherein the second composition further contains an adjuvant which is selected from the group comprising or consisting of Toll like receptor (TLR) agonists, NOD like receptor agonists, RIG-I like receptor agonists and STING pathway agonists, and mixtures of at least two of these.

9. Pharmaceutical combination of compositions for use in medical treatment according to claim 8, wherein the adjuvant contained in the second composition is selected from the group comprising a TLR1/2 agonist, a non-specific TLR3 agonist, a TLR4 agonist, a TLR5 agonist, a TLR6 agonist, a TLR7 agonist, a TLR8 agonist, a TLR9 agonist or a combination of at least two of these.

10. Pharmaceutical combination of compositions for use in medical treatment according to one of the preceding claims, wherein at least one of the portion of the target antigen and the co-stimulatory antibody effective for activating T-cells and/or dendritic cells, and optionally a compound effective for activating dendritic cells (DC) and/or T-cells, which compound is an adjuvant, of the second composition are contained in liposomes.

11. Pharmaceutical composition of compositions for use in medical treatment according to claim 10. wherein the liposomes containing the compounds of the second composition are cationic liposomes.

12. Pharmaceutical combination of compositions for use in medical treatment according to one of the preceding claims, wherein the non-specific TLR3 agonist is Poly(I:C) and/or PolyICLC or a homologue thereof, and/or wherein the STING pathway agonist is cdi-GMP.

13. Pharmaceutical combination of compositions for use in medical treatment according to one of the preceding claims, wherein the liposomes are unilamellar and have a size of 60 to 500 nm or of 140 to 180 nm.

14. Pharmaceutical combination of compositions for use in medical treatment according to one of the preceding claims, wherein the target antigen is a protein encoded by a nucleic acid under the control of genetic elements for synthesis of the encoded protein in a cell.

15. Pharmaceutical combination of compositions for use in medical treatment according to one of the preceding claims, wherein the medical treatment is for raising in a recipient a cellular immune response specifically directed against cells of the recipient bearing the target antigen.

16. Pharmaceutical combination of compositions for use in medical treatment according to one of claims 7 to 15, wherein the tumour is selected from the group comprising or consisting of hematological malignancies, Hodgkin and non-Hodgkin lymphomas, leukemias, especially acute lymphoblastic leukemia, acute myeloid leukemia, chronic lymphocytic leukemia, chronic myeloid leukemia, monocytic leukemia, myelomas, myeloproliferative diseases, myelodysplastic syndromes and solid cancers, especially originating from brain, head and neck, lung, pleura, heart, liver, kidney, colon, pancreas, stomach, gut, urinary tract, prostate, uterus, ovaries, breast, skin, testes, larynx and sarcoma.

17. Pharmaceutical combination of compositions for use in medical treatment according to one of the preceding claims, wherein the antigen is selected from the group consisting of tumour antigens, tumour neoantigens, tumour homogenate or tumour lysate.

18. Pharmaceutical combination of compositions for use in medical treatment according to one of the preceding claims, wherein the medical treatment comprises the generation of CD8+ T-cells which are specific for the target antigen and/or the generation of CD4+ T-cells which are specific for the target antigen.

19. Pharmaceutical combination of compositions for use in medical treatment according to one of the preceding claims, wherein the medical treatment generates activated CD8+ T-cells and/or CD4+ T-cells having specificity for autologous cells comprising the antigen.

20. Pharmaceutical combination of compositions for use in medical treatment according to one of the preceding claims, wherein the second composition is for administration one time only.

21. Pharmaceutical combination of compositions for use in medical treatment according to one of the preceding claims, wherein in the first composition and/or in the second composition the antigen is in soluble form.

## Patentansprüche

1. Pharmazeutische Kombination von Zusammensetzungen zur Verwendung bei der medizinischen Behandlung, wobei die Kombination eine erste Zusammensetzung umfasst, die Liposomen umfasst, die ein Zielantigen enthalten und zur Verabreichung wenigstens zwei Tage im Anschluss an die Verabreichung einer ersten Zusammensetzung eine zweite Zusammensetzung umfasst, die wenigstens einen Bereich des Zielantigens und einem co-stimulatorischen Antikörper, der zur Aktivierung von T-Zellen und/oder der dendritischen Zellen (DC) umfasst, wobei der co-stimulatorische Antikörper aus der Gruppe ausgewählt ist, die aus einem anti-CD137-Antikörper, einem anti-CD40-Antikörper, einem anti-OX 40 Antikörper, einem anti-ICOS Antikörper, einem anti-CD27 Antikörper, einem anti-CD28 Antikörper, einem anti-GITR Antikörper, insbesondere einem anti-Mensch-GITR/AITR-Antikörper, einem anti-HVEM-Antikörper, einem anti-CD TIM1-Antikörper, einem anti-TIM3-Antikörper und Mischungen von wenigstens zweien dieser besteht, und wobei die Zielantigene der ersten und der zweiten Zusammensetzung wenigstens ein für MHC I und/oder MHC II identisches Epitop enthalten.

2. Pharmazeutische Kombination von Zusammensetzungen zur Verwendung bei der medizinischen Behandlung nach Anspruch 1, wobei die Liposomen der ersten Zusammensetzung kationische Liposomen sind.

3. Pharmazeutische Kombination von Zusammensetzungen zur Verwendung bei der medizinischen Behandlung nach einem der voranstehenden Ansprüche, wobei die Liposomen eine zur Aktivierung dendritischer Zellen (DC) wirksame Verbindung enthalten, die einen Adjuvans ist, das aus der Gruppe ausgewählt ist, die Toll-Like-Rezeptoren (TLR)-Agonisten, NOD-Like-Rezeptor-Agonisten, RIG-I-Like-Rezeptor-Agonisten und STING-Weg-Agonisten und Mischungen von zumindest zweien dieser umfasst oder daraus besteht.

4. Pharmazeutische Kombination von Zusammensetzungen zur Verwendung bei der medizinischen Behandlung nach Anspruch 3, wobei der TLR-Agonist aus der Gruppe ausgewählt ist, die aus TLR 1/2-Agonisten, z. B. Lipoproteinen und/oder Peptidoglycanen, TLR3-Agonisten, z. B. doppelsträngiger RNA, Poly IC oder Poly ICLC, TLR4-Agonisten, z. B. Lipopolysacchariden, TLR5-Agonisten, z. B. Flagellin, TLR6-Agonisten, z. B. diacetylierte Lipoproteinen, TLR7-Agonisten und TLR8-Agonisten, z. B. einzelsträngiger RNA, TLR9-Agonisten, z. B. nicht-methylierte GpG-enthaltende DNA, TLR11-Agonisten, z. B. Profilin, und Kombinationen von zumindest zweien dieser besteht.

5. Pharmazeutische Kombination von Zusammensetzungen zur Verwendung bei der medizinischen Behandlung nach einem der Ansprüche 2 - 4, wobei die zur Aktivierung dendritischer Zellen (DC) wirksame Verbindung und/oder der LTR-Agonisten nur in den Liposomen eingeschlossen ist.

6. Pharmazeutische Kombination von Zusammensetzungen zur Verwendung in der medizinischen Behandlung nach einem der voranstehenden Ansprüche, wobei wenigstens eine der Verbindungen der zweiten Zusammensetzung in Liposomen formuliert ist und der co-stimulatorische Antikörper, der zur Aktivierung dendritischer Zellen (DC) und/oder T-Zellen wirksam ist und/oder das Adjuvans an der Außenseite der Liposomen und/oder nur innerhalb der Liposomen angeordnet ist.

7. Pharmazeutische Kombination von Zusammensetzungen zur Verwendung in der medizinischen Behandlung nach einem der voranstehenden Ansprüche, wobei die medizinische Behandlung die Behandlung von Tumoren, viraler Infektionen oder Infektionen durch intrazelluläre Bakterien ist.

8. Pharmazeutische Kombination von Zusammensetzungen zur Verwendung in der medizinischen Behandlung nach einem der voranstehenden Ansprüche, wobei die zweite Zusammensetzung weiterhin ein Adjuvans enthält, das aus der Gruppe ausgewählt ist, die Toll-Like-Rezeptor (TLR-Agonisten, NOD-Like-Rezeptor-Agonisten, RIG-I-Like-Rezeptor-Agonisten und STING-Weg-Agonisten und Mischungen von zumindest zweien dieser ausgewählt ist.

9. Pharmazeutische Kombination von Zusammensetzungen zur Verwendung in der medizinischen Behandlung nach Anspruch 8, wobei das in der zweiten Zusammensetzung enthaltene Adjuvans aus der Gruppe ausgewählt ist, die einen TLR1/2-Agonisten, einen nicht spezifischen TLR3-Agonisten, einen TLR4-Agonisten, einen TLR5-Agonisten, einen TLR6-Agonisten, einen TLR7-Agonisten, einen TLR5-Agonisten, einen TLR9-Agonisten oder eine Kombination von zumindest zweien dieser umfasst.

10. Pharmazeutische Kombination von Zusammensetzungen zur Verwendung in der medizinischen Behandlung nach einem der voranstehenden Ansprüche, wobei wenigstens ein Bereich des Zielantigens und des co-stimulatorischen Antikörpers, der zur Aktivierung von T-Zellen und/oder dendritischen Zellen wirksam ist, und optional eine Verbindung, die zur Aktivierung von den dendritischen Zellen (DC) und/oder T-Zellen wirksam ist, wobei diese Verbindung ein Adjuvans ist, der zweiten Zusammensetzung in Liposomen enthalten sind.

11. Pharmazeutische Kombination von Zusammensetzungen zur Verwendung bei der medizinischen Behandlung nach Anspruch 10, wobei die Liposomen, die die Verbindungen der zweiten Zusammensetzung enthalten, kationische Liposomen sind.

12. Pharmazeutische Kombination von Zusammensetzungen zur Verwendung in der medizinischen Behandlung nach einem der voranstehenden Ansprüche, wobei der nichtspezifische TLR3-Agonist Poly IC und/oder Poly ICLC oder ein Homolog davon ist, und/oder wobei der STING-Weg-Agonist cdi-GMP ist.

13. Pharmazeutische Kombination von Zusammensetzungen zur Verwendung in der medizinischen Behandlung nach einem der voranstehenden Ansprüche, wobei die Liposomen unilamellar sind und eine Größe von 60 bis 500 nm oder
von 140 bis 180 nm aufweisen.

14. Pharmazeutische Kombination von Zusammensetzungen zur Verwendung in der medizinischen Behandlung nach einem der voranstehenden Ansprüche, wobei das Zielantigen ein Protein ist, das von einer Nukleinsäure unter der Kontrolle eines genetischen Elements zur Synthese des codierten Proteins in einer Zelle ist.

15. Pharmazeutische Kombination von Zusammensetzungen zur Verwendung in der medizinischen Behandlung nach einem der voranstehenden Ansprüche, wobei die medizinische Behandlung eine zum Anheben einer spezifisch gegen Zellen des Empfängers, die das Zielantigen tragen, gerichteten zellulären Immunantwort in einem Empfänger ist.

16. Pharmazeutische Kombination von Zusammensetzungen zur Verwendung in der medizinischen Behandlung nach einem der Ansprüche 7 - 15, wobei der Tumor aus der Gruppe ausgewählt ist, die aus hämatologischen Malignitäten, Hodgkin und nicht-Hodgkin-Lymphomen, Leukämien, insbesondere akuter lymphomblastischer Leukämie, akuter myeloider Leukämie, chronischer lymphozytischer Leukämie, auch chronischer myeloider Leukämie, monozytischer Leukämie, Myelomen, myeloproliferativen Krankheiten, myelodysplastischen Syndromen und festen Krebsgeschwüren besteht, insbesondere solchen, die vom Gehirn, Kopf und Nacken, der Lunge, dem Zwerchfell, Herz, Leber, Niere, Darm, Pankreas, Magen, Verdauungstrakt, den Harnorganen, Prostata, Uterus, Eierstöcken, Brust, Haut, Hoden, Kehlkopf und Sarkom ausgeht.

17. Pharmazeutische Kombination von Zusammensetzungen zur Verwendung in der medizinischen Behandlung nach einem der voranstehenden Ansprüche, wobei das Antigen aus der Gruppe ausgewählt ist, die aus Tumorantigenen, Tumor-Neoantigenen, Tumorhomogenat oder Tumorlysat besteht.

18. Pharmazeutische Kombination von Zusammensetzungen zur Verwendung in der medizinischen Behandlung nach einem der voranstehenden Ansprüche, wobei die medizinische Behandlung das Erzeugen von CD 8+ T-Zellen umfasst, die für das Zielantigen spezifisch sind, und/oder die Erzeugung von CD 4+ T-Zellen, die für das Zielantigen spezifisch sind.

19. Pharmazeutische Kombination von Zusammensetzungen zur Verwendung in der medizinischen Behandlung nach einem der voranstehenden Ansprüche, wobei die medizinische Behandlung aktivierte CD 8+ T-Zellen und/oder CD 4+ T-Zellen erzeugt, die eine Spezifität für autologe Zellen aufweisen, die das Antigen umfassen.

20. Pharmazeutische Kombination von Zusammensetzungen zur Verwendung in der medizinischen Behandlung nach einem der voranstehenden Ansprüche, wobei die zweite Zusammensetzung nur zur einmaligen Verabreichung ist.

21. Pharmazeutische Kombination von Zusammensetzungen zur Verwendung in der medizinischen Behandlung nach einem der voranstehenden Ansprüche, wobei das Antigen in der ersten Zusammensetzung und/oder in der zweiten Zusammensetzung in löslicher Form ist.

## Revendications

1. Combinaison pharmaceutique de compositions pour utilisation dans un traitement médical, la combinaison comprenant une première composition comprenant des liposomes qui contiennent un antigène cible, et pour administration au moins 2 jours après l'administration de la première composition, une seconde composition comprenant au moins une partie de l'antigène cible et un anticorps co-stimulant efficace pour activer les cellules T et/ou les cellules dendritiques (DC), l'anticorps co-stimulant étant choisi dans le groupe comprenant un anticorps anti-CD137, un anticorps anti-CD40, un anticorps anti-OX40, un anticorps anti-ICOS, un anticorps anti-CD27, un anticorps anti-CD28, un anticorps anti-GITR, en particulier un anticorps anti-GITR/AITR humain, un anticorps anti-HVEM, un anticorps anti-TIM1, un anticorps anti-TIM3 et des mélanges d'au moins deux de ces anticorps, et dans lequel les antigènes cibles de la première composition et de la seconde composition contiennent au moins un épitope identique pour le CMH I et/ou pour le CMH II.

2. Combinaison pharmaceutique de compositions destinées à être utilisées dans un traitement médical selon la revendication 1, dans laquelle les liposomes de la première composition sont des liposomes cationiques.

3. Combinaison pharmaceutique de compositions destinées à être utilisées dans un traitement médical selon l'une des revendications précédentes, dans laquelle les liposomes contiennent un composé efficace pour activer les cellules dendritiques (CD) qui est un adjuvant choisi dans le groupe comprenant ou consistant en des agonistes des récepteurs Toll (TLR), des agonistes des récepteurs NOD, des agonistes des récepteurs RIG-I et des agonistes de la voie STING, et des mélanges d'au moins deux d'entre eux.

4. Combinaison pharmaceutique de compositions pour utilisation dans un traitement médical selon la revendication 3, dans laquelle l'agoniste TLR est choisi dans le groupe constitué des agonistes TLR1/2, par exemple les lipoprotéines et/ou les peptidoglycanes, les agonistes TLR3, par exemple l'ARN double brin, le Poly(I:C) ou le PolyICLC, les agonistes TLR4, par exemple les lipopolysaccharides, et les agonistes TLR3, par exemple les poly(I:C) ou les polyICLC, les agonistes TLR4, par exemple les lipopolysaccharides, et les poly(I:C). g. lipopolysaccharides, agonistes TLR5, p. ex. flagelline, agonistes TLR6, p. ex. lipoprotéines diacétylées, agonistes TLR7 et agonistes TLR8, p. ex. ARN simple brin, agonistes TLR9, p. ex. ADN non méthylé contenant des GpG, agonistes TLR11, p. ex. profiline, et combinaisons d'au moins deux de ces agonistes.

5. Combinaison pharmaceutique de compositions destinées à être utilisées dans un traitement médical selon l'une des revendications 3 à 4, dans laquelle le composé efficace pour activer les cellules dendritiques (DC) et/ou l'agoniste TLR est enfermé dans les liposomes uniquement.

6. Combinaison pharmaceutique de compositions destinées à être utilisées dans un traitement médical selon l'une des revendications précédentes, dans laquelle au moins l'un composés de la seconde composition est formuleé en liposomes, et l'anticorps co-stimulant efficace pour activer les cellules dendritiques (CD) et/ou les cellules T et/ou l'adjuvant est disposé à l'extérieur des liposomes et/ou à l'intérieur des liposomes uniquement.

7. Combinaison pharmaceutique de compositions pour utilisation dans un traitement médical selon l'une des revendications précédentes, dans laquelle le traitement médical est le traitement d'une tumeur, d'infections virales ou d'infections par des bactéries intracellulaires.

8. Combinaison pharmaceutique de compositions destinées à être utilisées dans un traitement médical selon l'une des revendications précédentes, dans laquelle la seconde composition contient en outre un adjuvant choisi dans le groupe comprenant ou consistant en des agonistes du récepteur Toll (TLR), des agonistes du récepteur NOD, des agonistes du récepteur RIG-I et des agonistes de la voie STING, et des mélanges d'au moins deux d'entre eux.

9. Combinaison pharmaceutique de compositions pour utilisation dans un traitement médical selon la revendication 8, dans laquelle l'adjuvant contenu dans la seconde composition est choisi dans le groupe comprenant un agoniste TLR1/2, un agoniste TLR3 non spécifique, un agoniste TLR4, un agoniste TLR5, un agoniste TLR6, un agoniste TLR7, un agoniste TLR8, un agoniste TLR9 ou une combinaison d'au moins deux de ces agonistes.

10. Combinaison pharmaceutique de compositions destinées à être utilisées dans un traitement médical selon l'une des revendications précédentes, dans laquelle au moins l'une des portions de l'antigène cible et de l'anticorps co-stimulant efficace pour activer les cellules T et/ou les cellules dendritiques, et éventuellement un composé efficace pour activer les cellules dendritiques (DC) et/ou les cellules T, lequel composé est un adjuvant, de la seconde composition sont contenus dans des liposomes.

11. Composition pharmaceutique de compositions destinées à être utilisées dans un traitement médical selon la revendication 10, dans laquelle les liposomes contenant les composés de la seconde composition sont des liposomes cationiques.

12. Combinaison pharmaceutique de compositions destinées à être utilisées dans un traitement médical selon l'une des revendications précédentes, dans laquelle l'agoniste TLR3 non spécifique est Poly(I:C) et/ou PolyICLC ou un homologue de ceux-ci, et/ou dans laquelle l'agoniste de la voie STING est le cdi-GMP.

13. Combinaison pharmaceutique de compositions à utiliser dans un traitement médical selon l'une des revendications précédentes, dans laquelle les liposomes sont unilamellaires et ont une taille de 60 à 500 nm ou de 140 à 180 nm.

14. Combinaison pharmaceutique de compositions destinées à être utilisées dans un traitement médical selon l'une des revendications précédentes, dans laquelle l'antigène cible est une protéine codée par un acide nucléique sous le contrôle d'éléments génétiques pour la synthèse de la protéine codée dans une cellule.

15. Combinaison pharmaceutique de compositions pour utilisation dans un traitement médical selon l'une des revendications précédentes, dans laquelle le traitement médical consiste à susciter chez un receveur une réponse immunitaire cellulaire spécifiquement dirigée contre les cellules du receveur portant l'antigène cible.

16. Combinaison pharmaceutique de compositions pour utilisation dans un traitement médical selon l'une des revendications 7 à 15, dans laquelle la tumeur est choisie dans le groupe comprenant ou consistant en des hémopathies malignes, des lymphomes hodgkiniens et non hodgkiniens, des leucémies, en particulier la leucémie lymphoblastique aiguë, la leucémie myéloïde aiguë, la leucémie lymphocytaire chronique, leucémie myéloïde chronique, leucémie monocytaire, myélomes, maladies myéloprolifératives, syndromes myélodysplasiques et cancers solides, notamment ceux du cerveau, de la tête et du cou, des poumons, de la plèvre, du coeur, du foie, des reins, du côlon, du pancréas, de l'estomac, de l'intestin, des voies urinaires, de la prostate, de l'utérus, des ovaires, du sein, de la peau, des testicules, du larynx et des sarcomes.

17. Combinaison pharmaceutique de compositions destinées à être utilisées dans un traitement médical selon l'une des revendications précédentes, dans laquelle l'antigène est choisi dans le groupe constitué par les antigènes tumoraux, les néoantigènes tumoraux, l'homogénat tumoral ou le lysat tumoral.

18. Combinaison pharmaceutique de compositions pour utilisation dans un traitement médical selon l'une des revendications précédentes, dans laquelle le traitement médical comprend la génération de cellules T CD8+ spécifiques de l'antigène cible et/ou la génération de cellules T CD4+ spécifiques de l'antigène cible.

19. Combinaison pharmaceutique de compositions à utiliser dans un traitement médical selon l'une des revendications précédentes, dans laquelle le traitement médical génère des cellules T CD8+ activées et/ou des cellules T CD4+ ayant une spécificité pour les cellules autologues comprenant l'antigène.

20. Combinaison pharmaceutique de compositions destinées à être utilisées dans un traitement médical selon l'une des revendications précédentes, dans laquelle la seconde composition est destinée à être administrée une seule fois.

21. Combinaison pharmaceutique de compositions destinées à être utilisées dans un traitement médical selon l'une des revendications précédentes, dans laquelle l'antigène est sous forme soluble dans la première composition et/ou dans la deuxième composition.
